# EUROPEAN PATENT SPECIFICATION

(11) **EP 4 463 566 B1**
(45) Date of publication and mention of the grant of the patent: **01.04.2026**
(21) Application number: 23709284.6
(22) Date of filing: 10.01.2023
(51) Int. Cl.: C12Q 1/6855, C12Q 1/6806

(54) **TARGET CAPTURE ULTRALONG-READ ANALYSIS**
ANALYSE MIT ULTRALANGEM LESEN EINER ZIELERFASSUNG
ANALYSE DE CAPTURE DE CIBLE ET DE LECTURE ULTRALONGUE

(30) Priority: 10.01.2022 US 202263297914 P
(43) Date of publication of application: 20.11.2024
(73) Proprietor: The Jackson Laboratory, Bar Harbor, ME 04609 (US)
(72) Inventor: WEI, Chia-Lin, Bar Harbor, ME 04609 (US); LESBIREL, Simon, Bar Harbor, ME 04609 (US); RECH, Gabriel, Bar Harbor, ME 04609 (US)
(74) Representative: Potter Clarkson
(86) International application number: PCT/US2023/010523
(87) International publication number: WO 2023/133359

(56) References cited:
- VAN HAASTEREN JOOST ET AL: "Genome-wide integration site detection using Cas9 enriched amplification-free long-range sequencing", NUCLEIC ACIDS RESEARCH, vol. 49, no. 3, 22 February 2021 (2021-02-22), GB, pages e16 - e16, XP093033541, ISSN: 0305-1048, Retrieved from the Internet <URL:https://www.ncbi.nlm.nih.gov/pmc/articles/PMC7897500/pdf/gkaa1152.pdf> DOI: 10.1093/nar/gkaa1152
- GOLDSMITH CHLOE ET AL: "Cas9-targeted nanopore sequencing reveals epigenetic heterogeneity after de novo assembly of native full-length hepatitis B virus genomes", MICROBIAL GENOMICS, vol. 7, no. 5, 18 May 2021 (2021-05-18), pages 507, XP093033102, Retrieved from the Internet <URL:https://www.ncbi.nlm.nih.gov/pmc/articles/PMC8209722/pdf/mgen-7-0507.pdf> DOI: 10.1099/mgen.0.000507
- GILPATRICK TIMOTHY ET AL: "Targeted nanopore sequencing with Cas9-guided adapter ligation", NATURE BIOTECHNOLOGY, vol. 38, no. 4, 1 April 2020 (2020-04-01), New York, pages 433 - 438, XP055853454, ISSN: 1087-0156, Retrieved from the Internet <URL:https://www.nature.com/articles/s41587-020-0407-5.pdf> DOI: 10.1038/s41587-020-0407-5
- HÖIJER IDA ET AL: "Amplification-free long-read sequencing reveals unforeseen CRISPR-Cas9 off-target activity", GENOME BIOLOGY, vol. 21, no. 1, 1 December 2020 (2020-12-01), XP093033106, Retrieved from the Internet <URL:https://genomebiology.biomedcentral.com/counter/pdf/10.1186/s13059-020-02206-w.pdf> DOI: 10.1186/s13059-020-02206-w
- HARRISON S. EDWARDS ET AL: "Real-Time Selective Sequencing with RUBRIC: Read Until with Basecall and Reference-Informed Criteria", SCIENTIFIC REPORTS, vol. 9, no. 1, 7 August 2019 (2019-08-07), XP055742888, DOI: 10.1038/s41598-019-47857-3

## Description

### BACKGROUND OF THE DISCLOSURE

### Field of the Disclosure

The invention, in some aspects, relates to methods and systems with which to identify genetic and epigenetic alterations in a target-specific manner.

Genetic analysis is limited by currently available methods. For example, mouse model validation remains dependent on Sanger sequencing or PCR based assays. Use of such methods to characterize animal models is very costly - and as a result only ~ 5% of transgenic mice have their insertion site known. Standard short-read sequencing approaches result in loss of structural data and are negatively impacted by the presence of repeat-rich regions and as such are inefficient and largely ineffective methods.

Goldsmith et al, 2021 (Microb. Genom., 7(5): 507. doi: 10. 1099/mgen.0.000507) relates to a method of Cas9-targeted nanopore sequencing for the detection of 5mCpG in the HBV genome that does not rely on bisulfite conversion or PCR. Gilpatrick et al, 2020 (Nat. Biotechnol., 38(4): 433-438. doi: 10.1038/s41587-020-0407-5) relates to a method of targeted nanopore sequencing with Cas9-guided adapter ligation. van Haasteren et al, 2021, Nucleic Acids Res, 49(3), e16 DOI: 10.1093/nar/gkaa1152 relates to extraction of fragments >50 kb which are fragmented by Cas9 cleavage, adapter ligated and sequenced to provide genetic and epigenetic information

### SUMMARY OF THE DISCLOSURE

The present invention relates to methods of identifying target-specific genetic and epigenetic information in a genomic region containing a DNA sequence of interest and methods of assessing efficacy of a means of introducing a candidate genetic modification in a cell. The present invention is defined in the accompanying claims. Embodiments of the invention are presented in the following numbered paragraphs:
1. A method of identifying target-specific genetic and epigenetic information in a genomic region containing a DNA sequence of interest, comprising:
   (a) extracting an ultralong DNA molecule from a biological sample, wherein the
      ultralong DNA molecule is at least 500 kb in length;
   (b) fragmenting the extracted ultralong DNA molecule to produce DNA molecule fragments, wherein one or more of the produced DNA molecule fragments comprises all or a portion of the DNA sequence of interest, and the cleaved fragments' ends are compatible for ligation of sequencing adaptors,
   (c) ligating the sequencing adaptors to the cleaved fragments, and
   (d) determining the sequences of the ligated cleaved fragments;

   wherein the determined sequences identify genetic and epigenetic information in the genomic region containing the DNA sequence of interest, and,
   wherein the method further comprises repeating the fragmenting in step (b) two or more times to cover the full sequence of interest.
2. The method of paragraph 1, wherein a method of determining the sequences comprises a nanopore sequencing means, comprising:
   (a) measuring an ionic current when a single-stranded DNA fragment of the extracted ultralong DNA molecule exposed to a voltage passes through a nanopore;
   (b) inferring a nucleotide sequence using real-time base calling from raw current signal data,
   (c) removing one or more unwanted DNA fragment molecules by reversing the voltage when the unwanted DNA fragment molecules pass across one or more individual nanopores; and
   (d) selecting the DNA fragment molecules containing the DNA sequence of interest.
3. The method of paragraph 1 or paragraph 2, wherein a means of fragmenting the ultralong DNA comprises an enzymatic method.
4. The method of any one of paragraphs 1-3, wherein the fragmenting means is a targeted-cleaving method.
5. The method of any one of paragraphs 1-4, wherein the biological sample comprises:
   (a) a cell,
      optionally wherein the DNA of interest is native to the cell or wherein the cell is a host cell and the DNA of interest is exogeneous DNA to the host cell, optionally wherein the exogenous DNA is:
      (i) DNA inserted into the host cell;
      (ii) episomal DNA in the host cell or DNA integrated into the host genome;
      (iii) a transgene in the host cell;
      (iv) comprising a unique sequence not present in the host cell's genome; or,
      (v) extrachromosomal; or
   (b) a body fluid, optionally wherein the body fluid comprises one of: blood, plasma, saliva, urine, lymph, amniotic fluid, cerebrospinal fluid.
6. The method of any one of paragraphs 1-5, wherein the DNA of interest comprises a predetermined DNA sequence or a DNA sequence positioned at preselected genomic coordinates obtained from a reference genome assembly.
7. The method of paragraph 4, wherein the targeted cleaving comprises:
   (i) binding a plurality of preselected Cas9 sgRNAs to the extracted ultralong DNA molecule, where the specificity of the binding is based on the DNA sequences of interest;
   (ii) contacting the bound preselected Cas9 sgRNAs with a plurality of one or more Cas9 enzymes; wherein the preselected Cas9 sgRNAs bound to the extracted ultralong DNA molecule each bind a Cas9 enzyme, forming a plurality of Cas9/sgRNA complex sites in the ultralong DNA molecule; and
   (iii) cutting the ultralong DNA molecule at the Cas9/sgRNA complexes thereby producing the DNA molecule fragments, wherein a number and position of the cuts are determined by the preselected Cas9 sgRNAs, and wherein a means for the cutting is a CRISPR/cas9 cutting method and the DNA fragments produced by the cutting comprise termini capable of ligation by the sequence adaptors.
8. The method of paragraph 4, wherein the targeted cleaving comprises:
   (i) binding a plurality of preselected Cas9 sgRNAs to the extracted ultralong DNA molecule;
   (ii) contacting the bound preselected Cas9 sgRNAs with a plurality of a non-endonuclease-deficient Cas9 enzyme and a plurality of an endonuclease-deficient Cas9 enzyme (dCas9); wherein the preselected Cas9 sgRNAs bound to the extracted ultralong DNA molecule each bind either a Cas9 enzyme or a dCas9 enzyme, forming Cas9/sgRNA complex sites and dCas9/sgRNA complex sites respectively in the ultralong DNA molecule; and
   (iii) cutting the ultralong DNA molecule at the Cas9/sgRNA complex producing the one or more DNA molecule fragments, wherein increasing a ratio of dCas9/sgRNA complex sites: Cas9/sgRNA complex sites in the ultralong DNA molecule decreases the number of cuts to the ultralong DNA molecule and the number of the produced DNA molecule fragments, and decreasing a ratio of dCas9/sgRNA complex sites: Cas9/sgRNA complex sites in the ultralong DNA molecule increases the number of cuts to the ultralong DNA molecule and the number of the produced DNA molecule fragments
   optionally further comprising preselecting the ratio of the dCas9/sgRNA complex sites: Cas9/sgRNA complex sites in the ultralong DNA molecule thereby preselecting a length of the produced DNA molecule fragments.
9. The method of paragraph 7 or paragraph 8, wherein:
   (a) the preselected sgRNAs bind the DNA sequences of interest;
   (b) the preselected sgRNAs are capable of binding a sequence contiguous with one or both ends of the DNA sequences of interest; or,
   (c) the preselected sgRNAs bind at one or more of: at positions (1) outside the DNA sequence of interest; (2) inside the DNA sequence of interest; and inside and outside the DNA sequence of interest.
10. The method of any one of paragraphs 1-9, further comprising ligating one or more sequencing adaptors to the ultralong DNA molecule fragments.
11. The method of any one of paragraphs 1-10, wherein a means of the sequencing comprises a nano pore sequencing method.
12. The method of any one of paragraphs 1-11, wherein the ultralong DNA molecule is between 1 kb and 500 kb in length, or wherein the ultralong DNA molecule is at least 500 kb in length.
13. The method of paragraph 1, wherein a means for the extracting comprises:
   (a) heating a preselected amount of cells to about 50-60°C in the presence of proteinase K and RNase A;
   (b) an alcohol-based precipitation of high molecular weight DNA; or,
   (c) a non-alcohol-based precipitation of high molecule weight DNA.
14. The method of any of paragraphs 1-13, further comprising comparing the determined ligated cleaved fragments with one or more reference sequence(s) and identifying a presence or absence of one or more differences in the determined ligated cleaved fragments and the reference sequences, wherein the comparison identifies one or more of a genetic alteration of: integration, insertion, deletion, inversion, translocations, and DNA modifications in the genomic region containing the DNA sequence of interest, optionally wherein the reference sequence comprises the genomic region containing the DNA sequence of interest in a wild-type cell.
15. The method of any one of paragraphs 5-14, wherein the cell from which the ultralong DNA molecule is extracted is:
   (a) a mammalian cell, optionally a mouse cell, further optionally wherein the mouse cell is a blood cell, optionally a plasma cell; or
   (b) a plant cell; or
   (c) from a mouse model of a disease or condition; or
   (d) a genetically engineered cell.
16. A method of assessing efficacy of a means of introducing a candidate genetic modification in a cell, the method comprising:
   assessing in a cell treated to introduce a candidate genetic modification in the cell, the sequences of the ligated cleaved fragments determined in paragraph 1(d); and
   identifying the presence or absence of the candidate genetic modification in the assessed determined sequences, wherein the presence of the candidate genetic modification confirms the efficacy of the means of introducing the candidate genetic modification in the cell.
17. The method of paragraph 16, wherein the cell is
   (a) a mammalian cell, optionally wherein the cell is from a mouse model of a disease or condition; or
   (b) a plant cell; or
   (c) a genetically engineered cell.

According to an aspect of the disclosure, a method of identifying target-specific genetic and epigenetic information in a genomic region containing a DNA sequence of interest is provided, the method including (a) extracting an ultralong DNA molecule from a biological sample, wherein the ultralong DNA molecule is at least 1 kb in length; (b) fragmenting the extracted ultralong DNA molecule to produce DNA molecule fragments, wherein one or more of the produced DNA molecule fragments comprises all or a portion of the DNA sequence of interest, and the cleaved fragments' ends are compatible for ligation of sequencing adaptors, (c) ligating the sequencing adaptors to the cleaved fragments, and (d) determining the sequences of the ligated cleaved fragments; wherein the determined sequences identify genetic and epigenetic information in the genomic region containing the DNA sequence of interest.

In some options, if the DNA sequence of interest is at least 500 kb in length, the method further comprises repeating the fragmenting in step (b) two or more times to cover the full sequence of interest. In certain options, a method of determining the sequences comprises a nanopore sequencing means, comprising: (a) measuring an ionic current when a single-stranded DNA fragment of the extracted ultralong DNA molecule exposed to a voltage passes through a nanopore; (b) inferring a nucleotide sequence using real-time base calling from raw current signal data, (c) removing one or more unwanted DNA fragment molecules by reversing the voltage when the unwanted DNA fragment molecules pass across one or more individual nanopores; and (d) selecting the DNA fragment molecules containing the DNA sequence of interest.

In certain options, a means of fragmenting the ultralong DNA comprises an enzymatic method. In some options, the fragmenting means is a targeted-cleaving method. In certain options, the biological sample comprises a cell. In certain options, the DNA of interest is native to the cell.

In some options, the cell is a host cell and the DNA of interest is exogeneous DNA to the host cell. In some options, the biological sample comprises a body fluid. In some options, the body fluid comprises one of: blood, plasma, saliva, urine, lymph, amniotic fluid, cerebrospinal fluid. In certain options, the DNA of interest comprises a predetermined DNA sequence or a DNA sequence positioned at preselected genomic coordinates obtained from a reference genome assembly.

In certain options, the exogenous DNA is DNA inserted into the host cell. In some options, the exogenous DNA of interest is episomal DNA in the host cell or DNA integrated into the host cell genome. In some options, the exogenous DNA is a transgene in the host cell. In certain options, the exogenous DNA comprises a unique sequence not present in the host cell's genome. In some options, in the exogenous DNA is extrachromosomal.

In some options, the targeted cleaving comprises: (i) binding a plurality of preselected Cas9 sgRNAs to the extracted ultralong DNA molecule, where the specificity of the binding is based on the DNA sequences of interest; (ii) contacting the bound preselected Cas9 sgRNAs with a plurality of one or more Cas9 enzymes; wherein the preselected Cas9 sgRNAs bound to the extracted ultralong DNA molecule each bind a Cas9 enzyme, forming a plurality of Cas9/sgRNA complex sites in the ultralong DNA molecule; and (iii) cutting the ultralong DNA molecule at the Cas9/sgRNA complexes thereby producing the DNA molecule fragments, wherein a number and position of the cuts are determined by the preselected Cas9 sgRNAs, and wherein a means for the cutting is a CRISPR/cas9 cutting method and the DNA fragments produced by the cutting comprise termini capable of ligation by the sequence adaptors.

In certain options, the targeted cleaving comprises: (i) binding a plurality of preselected Cas9 sgRNAs to the extracted ultralong DNA molecule; (ii) contacting the bound preselected Cas9 sgRNAs with a plurality of a non-endonuclease-deficient Cas9 enzyme and a plurality of an endonuclease-deficient Cas9 enzyme (dCas9); wherein the preselected Cas9 sgRNAs bound to the extracted ultralong DNA molecule each bind either a Cas9 enzyme or a dCas9 enzyme, forming Cas9/sgRNA complex sites and dCas9/sgRNA complex sites respectively in the ultralong DNA molecule; and (iii) cutting the ultralong DNA molecule at the Cas9/sgRNA complex producing the one or more DNA molecule fragments, wherein increasing a ratio of dCas9/sgRNA complex sites: Cas9/sgRNA complex sites in the ultralong DNA molecule decreases the number of cuts to the ultralong DNA molecule and the number of the produced DNA molecule fragments, and decreasing a ratio of dCas9/sgRNA complex sites : Cas9/sgRNA complex sites in the ultralong DNA molecule increases the number of cuts to the ultralong DNA molecule and the number of the produced DNA molecule fragments. In certain options, the method also includes preselecting the ratio of the dCas9/sgRNA complex sites : Cas9/sgRNA complex sites in the ultralong DNA molecule thereby preselecting a length of the produced DNA molecule fragments.

In some options, the preselected sgRNAs bind the DNA sequences of interest. In some options, the preselected sgRNAs are capable of binding a sequence contiguous with one or both ends of the DNA sequences of interest. In certain options, the preselected sgRNAs bind at one or more of: at positions (1) outside the DNA sequence of interest; (2) inside the DNA sequence of interest; and inside and outside the DNA sequence of interest. In certain options, the method also includes ligating one or more sequencing adaptors to the ultralong DNA molecule fragments. In some options, a means of the sequencing comprise a nanopore sequencing method. In some options, the ultralong DNA molecule is between 1 kb and 500 kb in length. In some options, the ultralong DNA molecule is at least 500 kb in length.

In certain options, a means for the extracting comprises heating a preselected amount of cells to about 50-60°C in the presence of proteinase K and RNase A. In certain options, a means for the extracting comprises an alcohol-based precipitation of high molecular weight DNA. In some options, a means for the extracting comprises a non-alcohol-based precipitation of high molecule weight DNA. In some options, the method also includes comparing the determined ligated cleaved fragments with one or more reference sequence(s) and identifying a presence or absence of one or more differences in the determined ligated cleaved fragments and the reference sequences, wherein the comparison identifies one or more of a genetic alteration of: integration, insertion, deletion, inversion, translocations, and DNA modifications in the genomic region containing the DNA sequence of interest. In certain options, the reference sequence comprises the genomic region containing the DNA sequence of interest in a wild-type cell.

In certain options, the cell from which the ultralong DNA molecule is extracted is a mammalian cell. In certain options, the cell from which the ultralong DNA molecule is extracted is a mouse cell. In some options, the mouse cell is a blood cell, optionally a plasma cell. In some options, the cell from which the ultralong DNA molecule is extracted is a plant cell. In certain options, the cell from which the ultralong DNA molecule is extracted is from a mouse model of a disease or condition. In some options, the cell from which the ultralong DNA molecule is extracted is a genetically engineered cell. In some options, the cell from which the ultralong DNA molecule is extracted from a genetically engineered animal or plant.

According to another aspect of the disclosure, a system for performing any embodiment of the aforementioned method of the disclosure is provided.

According to another aspect of the disclosure, a method of assessing efficacy of a means of introducing a candidate genetic modification in a cell is provided, the method including: assessing in a cell treated to introduce a candidate genetic modification in the cell, the sequences of the ligated cleaved fragments determined in any embodiment of an aforementioned method of the disclosure, and identifying the presence or absence of the candidate genetic modification in the assessed determined sequences, wherein the presence of the candidate genetic modification confirms the efficacy of the means of introducing the candidate genetic modification in the cell. In certain options, the cell is a mammalian cell. In certain options, the cell is a plant cell. In some options, the cell from a mouse model of a disease or condition. In some options, the cell is a genetically engineered cell. In certain options, the cell is obtained from a genetically modified animal or plant.

According to another aspect of the disclosure, a system for performing the method of any embodiment of the aforementioned method of the disclosure is provided.

According to another aspect of the disclosure, a method of assessing a genetic variation in a cell is provided, the method including: obtaining with any embodiment of an aforementioned method of the disclosure, genetic and epigenetic information in a genomic region containing a DNA sequence of interest; comparing the determined sequences of the ligated cleaved fragments determined in any embodiment of an aforementioned method of the disclosure, to one or more reference sequences and identifying presence or absence of one or more differences between the determined ligated cleaved fragment sequences and the reference sequences, wherein the presence of one or more difference(s) indicate a genetic variation in the cell. In certain options, the cell is known to have or is suspected of having a disease or condition and the reference sequence does not have the disease or condition. In some options, is obtained from a subject known to have or suspected of having the disease or condition. In some options, the method also includes assessing the genetic variation and its effect in the disease or condition.

According to another aspect of the disclosure, a method of assessing integration of an administered genetic material in a cell is provided, the method including; determining in the cell, with any embodiment of an aforementioned method of the disclosure, sequences of the ligated cleaved fragments of the DNA sequence of interest, wherein the cell comprises the administered genetic material and the administered genetic material comprises the DNA sequence of interest; comparing the sequences of the ligated cleaved fragments determined in any embodiment of an aforementioned method of the disclosure, to one or more reference sequences; and identifying based on the comparing, whether the administered genetic material is one or more of: not integrated in the cell, integrated episomally in the cell, and integrated into the genome of the cell. In certain options, the administered genetic material is administered to the cell in a vector. In some options, the vector is an adeno-associated virus (AAV) vector. In some options, the vector is a gene therapy vector. In certain options, the administered genetic material comprises therapeutic genetic material.

According to another aspect of the disclosure, a computer-implemented method of assessing a genetic variation in a DNA sequence of interest in a cell is provided, the computer-implemented method including: receiving data obtained with the method of any embodiment of an aforementioned method of the disclosure, wherein the data represents the sequences of the ligated cleaved fragments determined in any embodiment of an aforementioned method of the disclosure; processing, by at least one processor, the received data to assess the determined sequences; comparing, by the at least one processor, the determined sequences to reference sequences; and identifying, by the at least one processor, one or more differences between the determined sequences and the reference sequences, wherein the identified difference(s) indicate a genetic variation in the DNA sequence of interest in the cell. In certain options, the cell is known to have or is suspected of having a disease or condition and the reference sequence does not have the disease or condition.

In some options, the cell is obtained from a subject known to have or suspected of having the disease or condition. In some options, the computer-implemented method also includes assessing, by the at least one processor, the genetic variation and a potential effect of the variation in the cell. In some options, the comparing of the determined sequences to the reference sequences comprises identifying, by the at least one processor, one or more of a genetic alteration and a DNA modification in the genomic region containing the DNA sequence of interest. In certain options, the computer-implemented method also includes comparing, by the at least one processor, the determined sequences of the ligated cleaved fragments with one or more reference sequence(s); and identifying, by the at least one processor, a presence or absence of one or more differences in the determined sequences of the ligated cleaved fragments and the reference sequences, wherein the presence of one or more differences identifies one or more of a genetic alteration of: nucleotide substitution, insertion, deletion, inversion, translocations, and DNA modifications in the genomic region containing the DNA sequence of interest.

According to another aspect of the disclosure, a computer-implemented method of assessing integration of an administered genetic material in a cell is provided, the computer-implemented method including receiving data obtained with any embodiment of an aforementioned method of the disclosure, wherein the data represents the sequences of the ligated cleaved fragments determined in any embodiment of an aforementioned method of the disclosure, wherein the cell comprises administered genetic material and the administered genetic material comprises the DNA sequence of interest; processing the received data, by at least one processor, to compare the determined sequences of the ligated cleaved fragments to one or more reference sequences; and identifying, by the at least one processor and based on the comparing, whether the administered genetic material is one or more of: episomally in the cell or integrated into the genome of the cell. In certain options, the administered genetic material is administered to the cell in a vector. In some options, the vector is an adeno-associated virus (AAV) vector. In certain options, the vector is a gene therapy vector.

In certain options, the administered genetic material comprises therapeutic genetic material. In some options, the comparing of the determined ligated cleaved fragment sequences to the reference sequences comprises identifying, by the at least one processor, one or more of a genetic alteration and a DNA modification in the genomic region containing the DNA sequence of interest. In some options, the computer-implemented method also includes comparing, by the at least one processor, the selectively sequenced regions of the produced DNA molecule fragments of (ii) with one or more reference sequence(s); and identifying, by the at least one processor, a presence or absence of one or more differences in the selectively sequenced regions of the produced DNA molecule fragments and the reference sequences, wherein the comparison identifies one or more of a genetic alteration of: integration, insertion, deletion, inversion, translocations, and DNA modifications in the genomic regions containing the DNA sequence of interest.

According to another aspect of the disclosure, a computer-implemented method for identifying an integration event within a cell is provided, the computer-implemented method including receiving sequence data representing sequences of DNA of the cell, the sequences being determined using a nanopore sequencing technique; receiving an input representing that the received sequence data includes exogenous DNA; in response to receiving the input representing that the received sequence data includes exogenous DNA, mapping, by the at least one processor, the received sequence data to an indexed set of DNA sequences of interest, wherein the indexed set comprises sequences of at least one vector; identifying, by the at least one processor, portions of the received sequence data containing the DNA sequence of interest; mapping, by the at least one processor, the portions of the received sequence data containing the DNA sequence of interest to a reference genome using a long read mapper technique; and identifying, by the at least one processor, portions of the received sequence data with insertions embedded and the coordinate breakpoints in the reference genome, the identifying being performed using a structural variant identification technique and using the mapping of the portions of the received sequence data containing the DNA sequence of interest to the reference genome.

In certain options, the computer-implemented method also includes identifying, by the at least one processor, the portions of the received sequence data as hybrid sequences based on the portions aligning with the indexed set and the reference genome. In certain options, the computer-implemented method also includes reconstructing, by the at least one processor and using a de novo assembler, one or more inserted sequences from the portions of the received sequence data. In some options, the computer-implemented method also includes identifying, based on the reconstructed inserted sequence(s), in-tandem integration events within the cell. In some options, the computer-implemented method also includes identifying, based on the reconstructed inserted sequence(s), episomal sequences within the cell. In some options, the indexed set comprises one or more of: an Ad vector, a transgene, and a regulatory cassette.

According to another aspect of the disclosure, a computer-implemented method for identifying an integration event within a cell is provided, the computer-implemented method including receiving sequence data representing sequences of DNA of the cell, the sequences being determined using a nanopore sequencing technique; receiving an input representing that the received sequence data includes a reference genome sequence; in response to receiving the input representing that the received sequence data includes the reference genome sequence, mapping, by the at least one processor, the received sequence data to a reference genome using a long read mapper technique; determining, by the processor, sequence alignment data based on the mapping of the received sequence data to the reference genome; and identifying, by the at least one processor, portions of the received sequence data with structural variants based on the sequence alignment data, the identifying being performed using a structural variant identification technique. In certain options, identifying the structural variants comprises identifying single nucleotide polymorphisms (SNP) within the sequence alignment data. In certain options, the computer-implemented method also includes: identifying, by the at least one processor, a DNA modification within the sequence alignment data, the identifying being performed using a methylation technique.

These and other features, objects, and advantages of the present disclosure will become better understood from the description that follows. In the description, reference is made to the accompanying drawings, which form a part hereof and in which there is shown by way of illustration, not limitation, options of the disclosure. The description of preferred options is not intended to limit the invention to cover all modifications, equivalents, and alternatives. Reference should therefore be made to the claims recited herein for interpreting the scope of the invention.

### BRIEF DESCRIPTION OF THE DRAWINGS

The disclosure will be better understood and features, aspects, and advantages other than those set forth above will become apparent when consideration is given to the following detailed description thereof. Such detailed description refers to the following drawings.
**FIG. 1A-1B** show conceptual diagrams of a system for analyzing long-read sequencing data, according to the present disclosure.
**FIG. 2** shows a flowchart illustrating a process for characterizing a host genomic region expected to be, at least partially present in the reference genome of a cell, according to the present disclosure.
**FIG. 3** shows a flowchart illustrating another process for identifying integration events of an exogenous DNA within a cell, according to the present disclosure.
**FIG. 4** shows a block diagram conceptually illustrating example components of a device according to the present disclosure.
**FIG. 5** shows a block diagram conceptually illustrating example components of a server according to the present disclosure.
**FIG. 6** shows a schematic diagram illustrating the data analysis workflow for LORETA (LOng-REad TArgeted) pipeline according to the present disclosure.

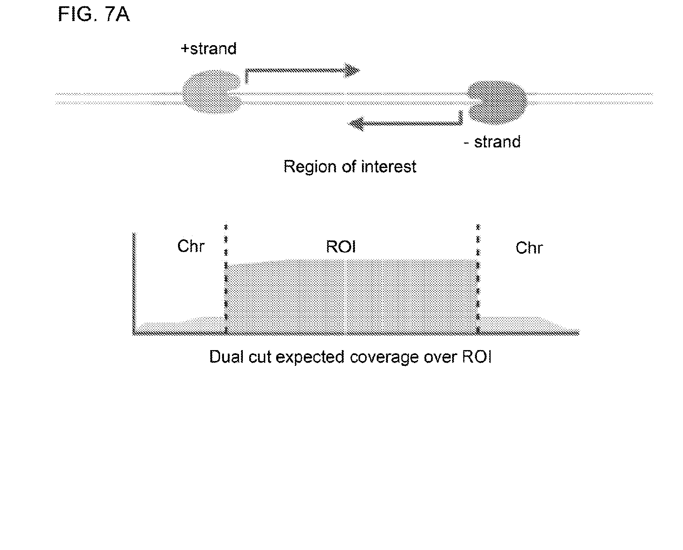

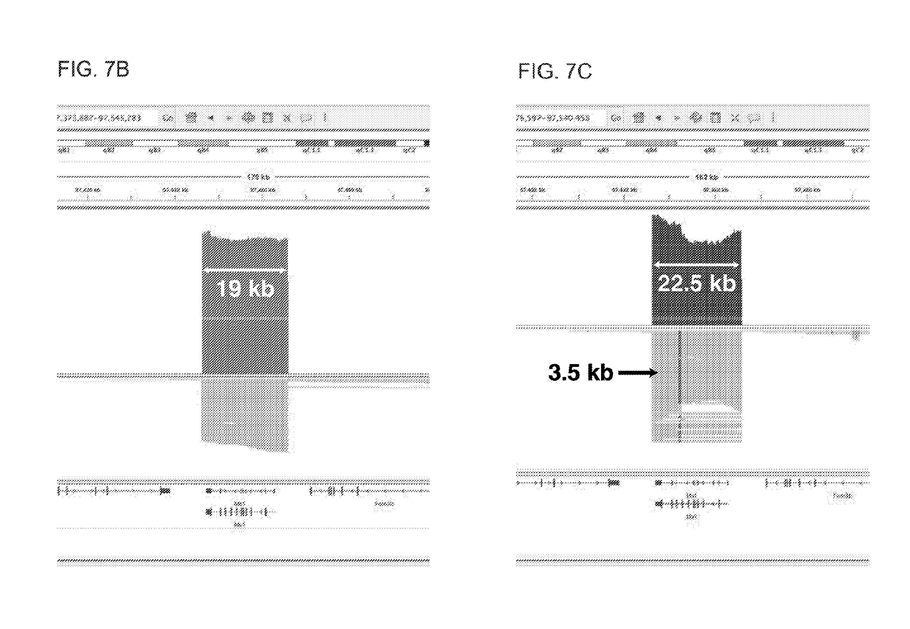

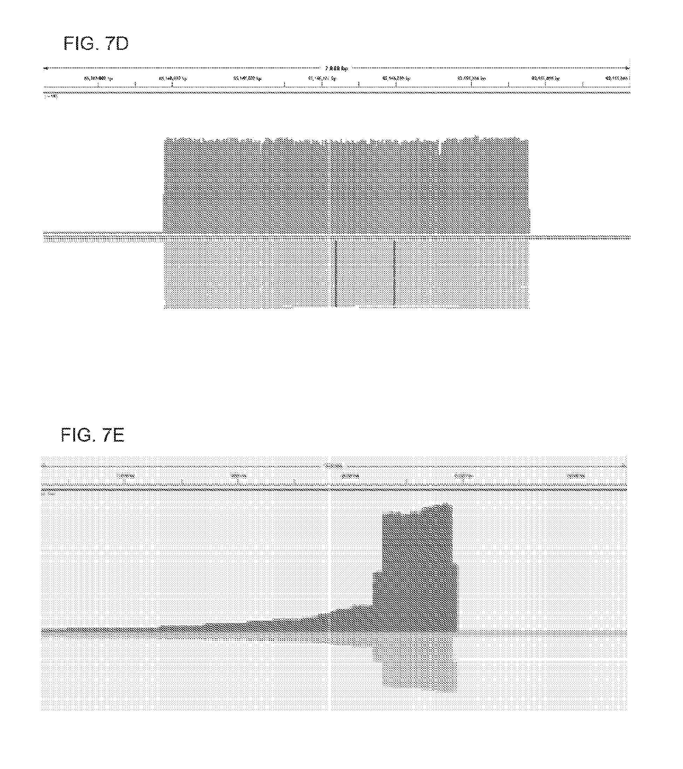

**FIG. 7A-7E** show a schematic diagram and images of sequence read alignments. FIG. 7A illustrates a dual cut Cas9 library design, with sgRNAs targeting 0.5-5kb upstream and downstream of a region or an insertion of interest. FIGS. 7B-C illustrate a comparison of the *MX1* locus in mouse lines C57BL/6J and CAST/EiJ. FIG. 7B shows alignments over a 19 kb region of C57BL/6J spanning the entire MX1 locus aligned to MM10. FIG. 7C shows alignments of a 22.5 kb region from CAST/EiJ spanning the MX1 locus and identifying (arrow) the 3.5 kb not present in C57BL/6J aligned to MM10. FIG. 7D shows alignments of a homozygous 5 kb region covering a floxed exon. FIG. 7E shows alignments of a 13 kb region of interest for validation of a targeted mutation.

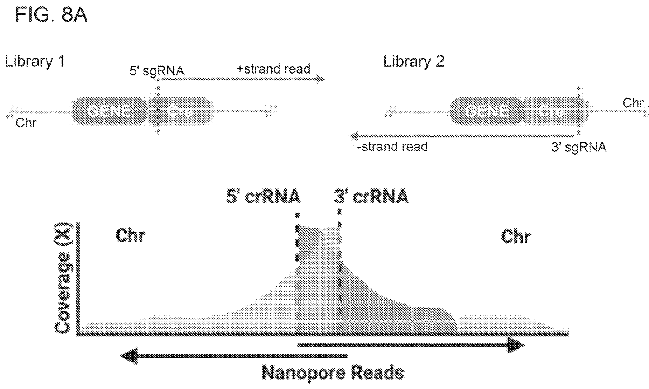

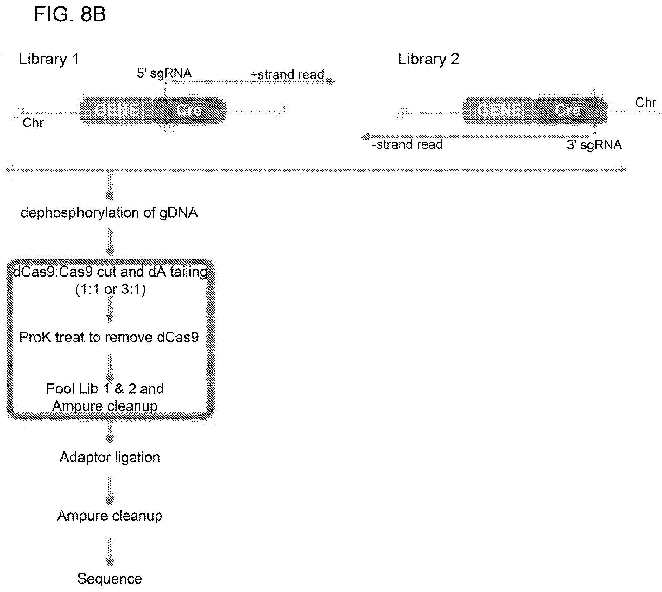

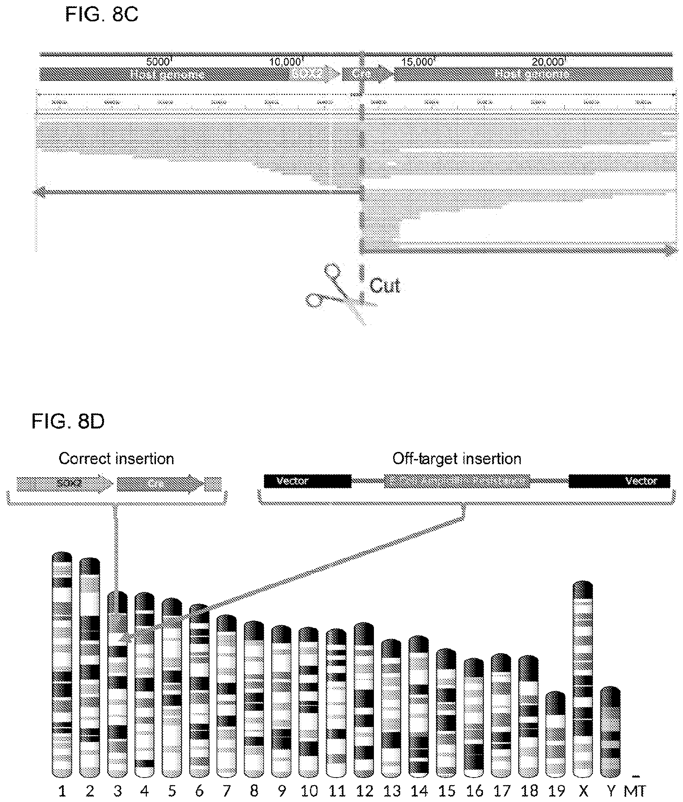

**FIG. 8A-8D** show schematic diagrams of single cut Cas9 library design for identifying insertions in unknown genome locations. FIG. 8A illustrates a strategy of targeting an inserted sequence and generating sequence reads beginning from the insertion and ending in the host chromosome. Dashed lines (upper and lower panels) represent sgRNAs targeted within an insertion. Red and yellow shaded areas (lower panel) represent read coverage from insertion sequence sites. FIG. 8B illustrates a single cut Cas9 library preparation workflow. The box indicates the additional steps for a dCas9 single cut library preparation workflow. FIG. 8C illustrates an example of validating a CRISPR-mediated modification using the single cut Cas9 library design strategy shown in FIG. 8A-8B. Each red or blue horizontal line represents an individual nanopore sequencing read. Red lines represent reads of library products obtained using a sense strand-targeting sgRNA; blue lines represent reads of library products obtained using an antisense strand-targeting sgRNA. FIG. 8D illustrates how on- and off-target insertions may be present in a genome, including on the same chromosome.

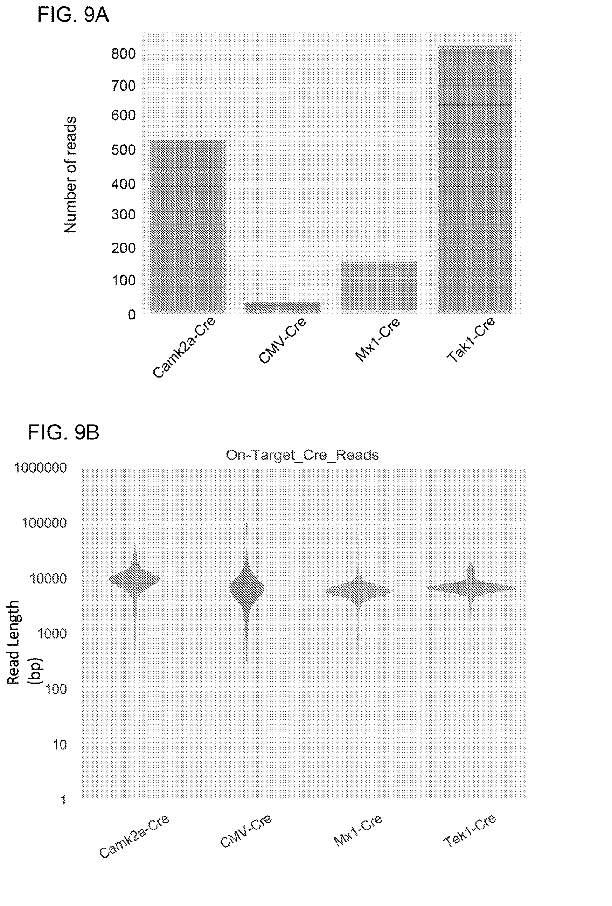

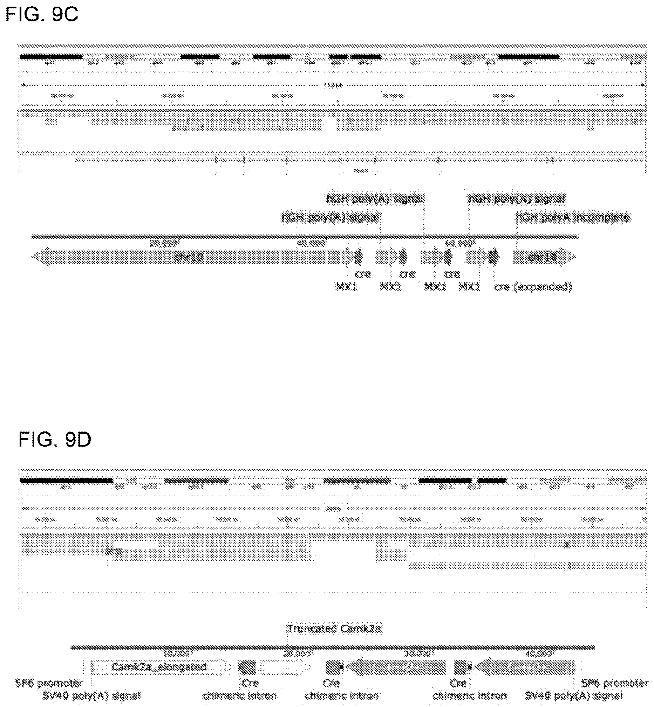

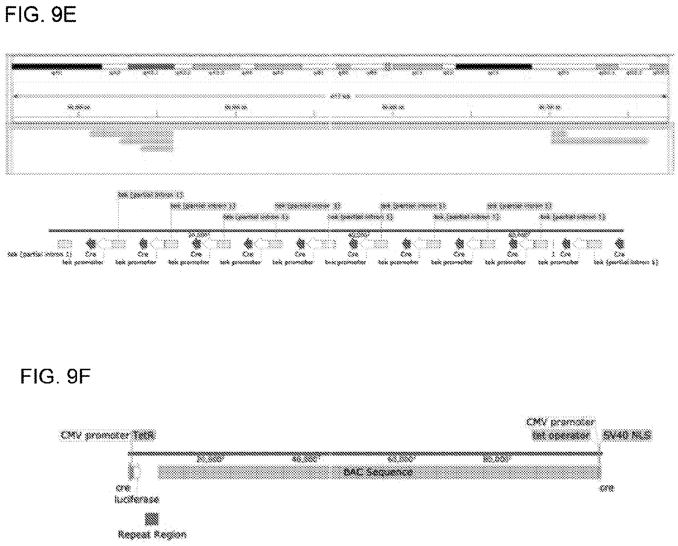

**FIG. 9A-9F** show schematic diagrams and graphs illustrating the application of a Cas9-targeted single-cut library strategy to identify genomic insert sites. FIG. 9A presents a bar graph showing the number of on-target Cre-containing reads in four mouse lines. FIG. 9B presents violin plots showing on-target read length distribution for the four mouse lines shown in FIG. 9A. Read length ranged from 4 to 100 kb. FIG. 9C (upper panel) shows identification of a MX1-Promoter-Cre transgene insertion region within the 5' region of Micu1 on Chr 10. FIG. 9C (lower panel) illustrates a reconstruction of the insertion region, revealing at least four copies of the MX1-Pro-Cre transgene. FIG. 9D (upper panel) shows identification of a Camk2a-Promoter-Cre transgene insertion region in Chr 17. FIG. 9D (lower panel) illustrates a reconstruction of the insertion region, revealing multiple integrations of the transgene cassette coupled with an inversion. FIG. 9E (upper panel) shows identification of a Tek-Promoter-Cre transgene insertion region on Chr 13. FIG. 9E (lower panel) illustrates a reconstruction of the insertion region, revealing at least 12 copies of the transgene cassette. FIG. 9F shows a schematic illustrating identification of an unwanted integration: an 80kb fragment of a BAC plasmid backbone along with an hGH-Promoter-Cre transgene integration in Chr X.
**FIG. 10A-10C** show schematic diagrams and a graph illustrating a dCas9 single-cut library strategy. FIG. 10A presents a schematic diagram depicting concatemerization of a transgene insertion and the resulting generation of short reads resulting in over-representation of the individual cassette in sequencing data. FIG. 10B illustrates suppression of short read generation from a concatemer through the addition of dCas9. FIG. 10C shows violin plots illustrating results of using dCas9 to reduce Cas9 cutting and increase read length, thereby removing individual cassette bias in sequencing data.

### DETAILED DESCRIPTION

Methods and systems of the disclosure provide an end-to-end solution to analyze genetic and epigenetic alterations at target-specific manner. Use of options of methods and systems enable precise and complete characterization of chromosomal target regions or genetic elements of interest and can be used to assess genetic and epigenetic status of any organism with reference genome sequence available. Methods and systems of the disclosure comprise combinations of elements such as an optimized ultralong DNA extraction, CRISPR-based genome targeting, and sequencing methods to generate ultralong read sequences from target regions of different sizes. In certain options, methods and systems of the disclosure include a specialized computational analytic pipeline, and can achieve high resolution, cost efficiency, fast turnaround, and comprehensive analysis of the genetic configuration from any regions of interest from the genomes. Non-limiting examples of applications for options of methods and systems of the disclosure include full spectrum transgene integration and genetic editing characterization, analysis of transgenic animal models, potential genotoxic integration events in clinical trials of gene therapy, potential off-target effects for gene therapy studies, detection of clinically relevant genomic alterations for diagnostic purpose, targeted molecular karyotyping, screening for known mutations in population of predisposition, and novel discovery of new genetic variants.

Aspects of the disclosure provide methods and systems to obtain target-specific genetic and epigenetic information. A targeted long-read genetic methods analysis platform of the disclosure provides an end-to-end system (from sample to interpretation) with advantages over prior methods including in areas such as: precision (base pair accuracy); comprehensiveness (targeting 100s to Mb sizes); efficiency (low cost and fast turn-around); and quality (high target enrichment rate). Non-limiting examples of how options of long-read methods and systems of the disclosure can be used in methods such as, but not limited to identification of detailed structure of genomic regions, insertion sites for random transgenics, off-target integration for CRISPR generated models and vector integrations, identity and integrity. Another feature of methods and systems of the disclosure is the ability to use a wide range of starting materials, non-limiting examples of which are: cells, tissues, blood samples, fluid samples, etc.

Options of methods and systems of the disclosure are useful in clinical applications, non-limiting examples include: diagnostic methods, targeted molecular karyotyping, identification of clinically-relevant chromosomal alteration in genetic disease of unknown origins and to validate artificially engineered loci. Certain options of methods and systems of the disclosure may be used in regulatory surveillance of clinical procedures, a non-limiting example of which is for assessing gene therapy procedures. For example, methods and systems of the disclosure can be used to identify the presence or absence of an off-target effect for gene therapy which provides safety and quality control process information. Thus, methods of the disclosure can be used to assess efficacy and safety of gene therapies in subjects. In addition, certain options of methods and systems of the disclosure can be used as research tools, non-limiting examples of which are their use for full spectrum transgene integration characterization, standard genomic analysis, and assessment of transgenic animal and/or plant models. Targeted long-read sequencing methods and systems of the disclosure can also be used to characterize repetitive regions of the genome such as satellite regions, tandem repeat expansions/contractions and transposable elements.

### Long-read Analysis

As described herein, options of methods of the disclosure include long-read target-specific sequence determinations. The disclosure, in part, also includes a system capable of performing options of method of the long-read target specific sequence determination as described herein. In some options the disclosure is capable of identifying, in a targeted-specific manner, genetic and epigenetic information in a genomic region containing a DNA sequence of interest. As used herein the term "DNA sequence of interest" means a target sequence about which the practitioner desires information about presence or absence, amount, structure, location, modification and other genetically relevant information. As a non-limiting example, a sequence of interest in some options may be a sequence administered as part of a gene therapy and methods of the disclosure are used to identify if, after administration of the gene therapy to a subject, the sequence of interest is present or absent in cells of the subject, is inserted in the genome of cells intended to include the sequence; is present in off-target cells or genomic regions, etc. In another non-limiting example, a sequence of interest is a gene sequence and methods of the disclosure are used to identify the sequence of interest and determine the presence or absence of differences between the identified sequence and a reference sequence for that gene. As used herein the term "reference sequence" is a control sequence, a non-limiting example of which is a wild-type sequence. A reference sequence as used herein is a sequence that can serve as a baseline sequence and an identified sequence of interest can be compared to its reference sequence and differences in the compared sequences identified.

Sequence of interest is the DNA sequence under study, a.k.a. the targeted sequence that is under study (subject of the analysis) either because it has potential clinical implications or is the object of a genetic editing characterization (among others). Non-limiting examples of a sequence of interest are: selected sets of clinically relevant genes or genomic regions; or gene delivery vehicles such as viral vectors (e.g. AAV/Lenti); and transgenic constructions (e.g. Promoter+Cre, Promoter+GFP, Gene +Cre, Gene+GFP).

In some options the methods includes extracting an ultralong DNA molecule from a biological sample. As used herein, the term "ultralong" means at least 1 kb in length. An ultralong DNA molecule may be about 1 kb, between 1 kb and 100 kb; between 1 kb and 500 kb, between 1 kb and 1000 kb, between 100 kb and 500 kb, between 100 kb and 1000 kb, between 500 kb and 1000 kb in length (inclusive). In some options an ultralong DNA molecule is greater than 500 kb in length.

In some options a means for extracting the ultralong DNA from a biological sample is an enzymatic extraction comprising heating a preselected amount of cells of the biological sample in the presence of a proteinase. A non-limiting example includes heating cells of a biological sample to about 50-60oC in the presence of proteinase K and RNase A. In certain options of methods and systems of the disclosure, the extraction means comprises an alcohol-based precipitation of high molecular weight. DNA from the biological sample. In some options a means for the extracting comprises a non-alcohol-based precipitation of high molecule weight DNA from the biological sample. Additional non-limiting examples of extraction methods include: DNA extraction techniques include organic extraction (phenol-chloroform method), nonorganic method (salting out and proteinase K treatment), and adsorption method (silica-gel membrane), see for example Phenol-Chloroform Extraction Trends in Food Science & Technology. It will be understood that alternative means of extracting ultralong DNA may also be used in certain methods and systems of the disclosure.

Following extraction of the ultralong DNA molecules, methods of the disclosure include fragmenting the extracted ultralong DNA molecule thereby producing DNA molecule fragments. The fragmenting results in one or more produced DNA molecule fragments that comprise all or a portion of the DNA sequence of interest. In certain options of methods and systems of the disclosure, the extracted DNA ultralong sequence fragmented with one fragmenting step may result in fragments that include the entirety of the sequence of interest in the generated fragments. In some options of methods and systems of the disclosure, an extracted DNA ultralong sequence fragmented with one fragmenting step may result in fragments that do not include entirety of the sequence of interest in the generated fragments. In this instance the fragmenting may be repeated one or more times, which results in presence of the entire sequence of interest in the totality of the produced DNA molecule fragments. In options in which a only portion of the entirety of the DNA sequence of interest is included in the produced DNA molecule fragments, the fragmenting step may be carried out two or more times, to result in presence of the entire sequence of interest in the resulting fragments.

Whether to include one fragmenting step or to include two or more fragmenting steps in a method or system of the disclosure may be based at least in part on the length of the DNA sequence of interest. For example, if the sequence of interest is at least 500 kb in length two or more fragmenting steps may be included in the method and/or system. In some instances additional fragmenting steps are included if the sequence of interest is at least 400 kb in length, at least 500 kb in length, at least 600 kb in length, at least 700 kb in length, at least 800 kb in length, at least 900 kb in length, or at least 1000 kb in length. As a non-limiting example, a sequence of interest in an extracted ultralong DNA molecule is about 300 kb in length. In this instance the ultralong DNA undergoes one fragmenting step, and all of the sequence of interest is present in the totality of the one or more produced DNA molecule fragments. As another non-limiting example, a sequence of interest in another extracted ultralong DNA is about 600 kb in length. In this instance the ultralong DNA undergoes two or more fragmenting steps and as a result all of the sequence of interest is present in the totality of the produced DNA molecule fragments.

Non-limited examples of means for fragmenting an ultralong DNA molecule in a method and/or system of the disclosure are enzymatic methods and targeted-cleaving methods. It will be understood that members of the Cas family with nuclease/cleavage activity can be used in methods and systems of the disclosure. See Nidhi,, S. et al., Int J Mol Sci. 2021 Apr; 22(7): 3327.

### Targeted Cleaving

Methods and systems may include targeted cleaving of the ultralong DNA molecule. In some options of methods and systems of the disclosure targeted cleaving includes use of CRISPR-Cas9 cleavage methods. CRISPR-Cas9 methods are known in the art as capable of RNA-guided genome editing and transcription regulation in applications such as targeted genome modification and site-directed mutagenesis. In certain options of methods and systems of the disclosure, Cas9 cleavage is used to cleave the ultralong DNA molecule. Cas9 cleavage methods comprise use of the Cas9 protein and guide RNA (sgRNAs) and in certain options, one or more sgRNAs are preselected to be capable of binding the DNA sequence of interest. Cas9 and the sgRNAs interact with each other and form a complex that identifies specific target sequences with high selectivity. The Cas9 protein locates and cleaves the targeted DNA at the location of the sgRNA binding. Thus, one or more sgRNA may be preselected for use in a method of the disclosure based at least in part on the sequence of interest and the ability of the sgRNA to bind to the sequence of interest. In some options of methods of the disclosure, preselected sgRNAs are selected because of the location on the DNA of interest on at which the sgRNA binds. In a non-limiting example, a preselected sgRNAs is selected for use in an embodiment of a method and/or system of the disclosure, because the sgRNA is capable of binding a sequence contiguous with one or both ends of the DNA sequence of interest. It will be understood that a preselected sgRNA may be selected at least in part because it is capable of binding at (1) a position on the ultralong DNA molecule outside the DNA sequence of interest; (2) a position inside the DNA sequence of interest; and/or (3) a position in the DNA sequence of interest and at a position on the ultralong-DNA molecule outside the DNA sequence of interest.

Certain options of targeted cleaving may include steps of: (i) binding a plurality of preselected Cas9 sgRNAs to the extracted ultralong DNA molecule, where the specificity of the binding is based on the DNA sequences of interest, then (ii) contacting the bound preselected Cas9 sgRNAs with a plurality of one or more Cas9 enzymes; wherein the preselected Cas9 sgRNAs bound to the extracted ultralong DNA molecule each bind a Cas9 enzyme, forming a plurality of Cas9/sgRNA complex sites in the ultralong DNA molecule; and (iii) cutting the ultralong DNA molecule at the Cas9/sgRNA complexes. The cutting results in DNA molecule fragments, wherein a number and position of the cuts in the sequence are determined by the preselected Cas9 sgRNAs. In some options of targeted cleaving, a means for the cutting is a CRISPR/cas9 cutting method and the DNA fragments produced by the cutting comprise termini capable of ligation by the sequence adaptors.

In other options of the disclosure targeted cleaving includes steps of (i) binding a plurality of preselected Cas9 sgRNAs to the extracted ultralong DNA molecule; (ii) contacting the bound preselected Cas9 sgRNAs with a plurality of a non-endonuclease-deficient Cas9 enzyme and a plurality of an endonuclease-deficient Cas9 enzyme (dCas9). In this embodiment, the preselected Cas9 sgRNAs bound to the extracted ultralong DNA molecule each bind either a Cas9 enzyme or a dCas9 enzyme, which forms Cas9/sgRNA complex sites and dCas9/sgRNA complex sites respectively in the ultralong DNA molecule. When the ultralong DNA molecule is then cut, the cutting occurs at the Cas9/sgRNA complex sites and not at the dCas9/sgRNA complex sites. Cutting the ultralong DNA molecule at the Cas9/sgRNA complex produces the one or more DNA molecule fragments, and the number and size of the fragments can be determined by the ratio or dCas9/sgRNA sites to Cas9/sgRNA sites. For example, increasing the ratio of dCas9/sgRNA complex sites: Cas9/sgRNA complex sites in the ultralong DNA molecule results in a decrease in the number of cuts to the ultralong DNA molecule and the number of the produced DNA molecule fragments. Decreasing the ratio of dCas9/sgRNA complex sites : Cas9/sgRNA complex sites in the ultralong DNA molecule results in an increase in the number of cuts to the ultralong DNA molecule and the number of the produced DNA molecule fragments. Some options of methods and systems of the disclosure, also include preselecting a ratio of the dCas9/sgRNA complex sites : Cas9/sgRNA complex sites in the ultralong DNA molecule as a means of preselecting a length of the produced DNA molecule fragments. Thus, selecting the ratio is used in certain options of methods of the disclosure to predetermine the number of cuts and therefore the length of the produce DNA molecule fragments. Thus, a higher ratio of dCas9/sgRNA to Cas9/sgRNA results in fewer cuts and longer produced DNA molecule fragments compared to a lower ratio of dCAS9/sgRNA to Cas9/sgRNA, which results in more cuts and shorter produced DNA molecule fragments.

In some options of a method and/or a system of the disclosure the ratio of dCas9/sgRNA complex sites : Cas9/sgRNA complex sites is: 1:1, 1:2, 2:1, 1:3, 3:1, 1:4; 4:1, 1:5, or 5:1. In some options of methods of the disclosure, a means for the cutting is a CRISPR/cas9 cutting method and the DNA fragments produced by the cutting comprise termini capable of ligation by the sequence adaptors. As a non-limiting example, a Cas9 enzyme is used to cut an ultralong DNA molecule and the ends of the resulting cleaved fragments are compatible for ligation of sequencing adaptors. Sequencing methods that can be used in methods and systems of the disclosure, including but not limited to methods that result in cleaved fragments compatible for ligation of sequencing adaptors are known in the art, see Gilpatrick, T, et al., Nat Biotechnol 38, 433-438 (2020). In some options of methods and systems of the disclosure the sequencing adaptors are ligated to the ends of the cleaved fragments, and the sequences of the ligated cleaved fragments are determined. Sequencing may be carried out using standard methods, or may be carried out using a method comprising a nanopore sequencing means. If a nanopore sequencing means is included in a method and/or system of the disclosure, the nanopore sequencing means comprises measuring an ionic current when a single-stranded DNA fragment of the extracted ultralong DNA molecule exposed to a voltage passes through a nanopore; inferring a nucleotide sequence using real-time base calling from raw current signal data, removing one or more unwanted DNA fragment molecules by reversing the voltage when the unwanted DNA fragment molecules pass across one or more individual nanopores; and selecting the DNA fragment molecules containing the DNA sequence of interest. Nanopore methods are known in the art and it will be understood how a nanopore method can be carried out in conjunction with a method and/or system of the disclosure. See for example: Gilpatrick, T, et al., Nat Biotechnol 38, 433-438 (2020) and Wang, Y. et al., Nature Biotechnol. 39, 1348-1365 (2001).

### DNA Sequence of Interest

As described herein, the terms "sequence of interest" or "DNA of interest" which may be used interchangeably herein, means a target sequence about which a practitioner desires information about one or more of: the nucleotide sequence of a nucleic acid (RNA or DNA); presence or absence of the nucleic acid sequence, for example though not intended to be limiting, presence or absence in a cell obtained from a biological sample; an amount of the DNA sequence, a structure of the DNA sequence molecule; a location of the DNA sequence, for example, though not intended to be limiting, as a chromosomal sequence or an episomal DNA sequence; and/or other physical and/or spatial information. In some options of methods and/or systems of the disclosure a DNA of interest comprises a predetermined DNA sequence or a DNA sequence positioned at preselected genomic coordinates obtained from a reference genome assembly. The positioning of the DNA sequence is determined by either, but not limited to, the information provided by publicly available genome browsers or by mapping the DNA sequence to the reference genome based on sequence identity.

Some options of methods and systems of the disclosure, include identifying target-specific genetic and epigenetic information in a cell. The cell may be present in a biological sample from which an ultralong DNA molecule is extracted as described above herein, meaning the ultralong DNA molecule is extracted from a cell present in the biological sample. In some options a DNA of interest is endogenous to the cell from which it is extracted, meaning it is native and naturally occurring in that cell. In certain options of methods and systems of the disclosure, a DNA of interest is an exogenous DNA that is not naturally occurring in the cell, which, in some instances means the exogenous DNA comprises a unique sequence not present in the host cell's genome. As a non-limiting example, an exogenous DNA may be DNA that has been inserted into the cell, which may also be referred to herein as a "host cell." In some options, an exogenous DNA of interest is episomal DNA in the host cell or DNA that has integrated into the host cell genome. An exogenous DNA may in some options be a transgene in a host cell and in some options of methods and systems of the disclosure, a DNA of interest is extrachromosomal DNA (ecDNA).

### Reference Sequences

In certain options of methods and systems of the disclosure, a sequence determined for a ligated cleaved fragment of the DNA of interest is compared to one or more reference sequences. As used herein the term "reference sequence" is a sequence that serves as a control sequence and a determined sequence can be compared against the reference sequence to identify similarities and/or differences between the determined sequence and the reference sequence. A determined sequence of a ligated cleaved fragment of the DNA may be compared against one or more reference sequence(s) thereby resulting in identification of a presence or absence of one or more differences in the determined ligated cleaved fragments and the reference sequences. A comparison of a determined sequence of a ligated cleaved fragment of the DNA to a reference sequence may result in identification of one or more of a genetic alteration of: integration, insertion, deletion, inversion, translocations, and one or more DNA modifications in the genomic region containing the DNA sequence of interest. In some options, a reference sequence includes the genomic region in a wild-type cell, wherein the genomic region contains the DNA sequence of interest. Another non-limiting example of a reference sequence may be a sequence that includes the genomic region in a genetically engineered cell wherein the genomic region contains the DNA sequence of interest. In some options, a reference sequence is a sequence that includes the genomic region in a cell with a disease or condition, wherein the genomic region contains the DNA sequence of interest. In some options, a sequence may be determined for a ligated cleaved fragment of a DNA of interest obtained from a cell that has been contacted with a candidate therapeutic and a reference sequence may be a sequence of the DNA of interest of a cell not contacted with the candidate therapeutic. In certain options of methods and systems of the disclosure, a candidate therapeutic is a gene therapy agent and a cell contacted with the gene therapy agent is assessed using a method of the disclosure and the resulting determined sequence of a ligated cleaved fragment of the DNA of interest can be compared to a reference or control sequence to determine the efficacy of the administered gene therapeutic agent.

### Biological Samples and Cells

In certain options of methods and systems of the disclosure, an ultralong DNA sequence is extracted from a biological sample. As used herein the term "biological sample" means a sample comprising a cell or cells. A biological sample used in a methods or system of the disclosure may be obtained from a living subject, a deceased subject, cell culture, organ culture, or tissue culture. In some options, a biological sample comprises a body fluid. Non-limiting examples of one or more body fluids that may be included in a biological sample are: blood, plasma, saliva, urine, lymph, amniotic fluid, and cerebrospinal fluid. A cell in a biological sample may be a normal, non-diseased cell. In some options of methods and systems of the disclosure, a cell is in a biological sample obtained from a subject who has, or is suspected of having a disease or condition. In some options of methods and systems of the disclosure, a cell in a biological sample is an engineered cell. In some options, a cell in a biological sample is a host cell, into which DNA exogenous to the cell has been inserted.

Non-limiting examples of cells that may be used in an embodiment of a method of the disclosure are one or more of: rodent cells, dog cells, cat cells, avian cells, fish cells, plant cells, cells obtained from a wild animal, cells obtained from a domesticated animal, and other suitable cell of interest. A cell that may be used in certain options of the disclosure is a human cell. In some options a cell is a stem cell, an embryonic stem cell, or embryonic stem cell-like cell. In some options of the disclosure a cell is a naturally occurring cell and in certain options of the disclosure a cell is an engineered cell.

In some options of methods and systems of the disclosure, a cell from which the ultralong DNA molecule is extracted is a mammalian cell. In some options, mammalian cell from which the ultralong DNA molecule is extracted is a mouse cell. In certain options of methods and systems of the disclosure, a mammalian cell is a blood cell, optionally a plasma cell. In some options of a method and/or system of the disclosure, the cell from which the ultralong DNA molecule is extracted is from a mouse model of a disease or condition. In certain options of methods and/or systems of the disclosure the cell from which the ultralong DNA molecule is extracted is a genetically engineered cell.

It will be understood that cells or a cell sample used in a method of the disclosure comprises a plurality of cells. As used herein the term "plurality" means more than one. In some instances a plurality of cells is least 1, 10, 100, 1,000, 10,000, 100,000, 500,000, 1,000,000, 5,000,000, or more cells. A plurality of cells included in a sample may be a population of cells. A plurality of cells may include cells that are of the same cell type. In some options of the disclosure, a plurality of cells includes cells having a known or suspected disease or condition. In some options of the disclosure, a plurality of cells is a mixed population of cells, meaning the cells are not all of the same cell type. A cell used in a method of the disclosure, may be obtained from a biological sample obtained directly from a subject. In some options, cells are obtained from surgical specimens, tissue or cell biopsies, etc. Non-limiting examples of biological samples are samples of: tissue, skin, cartilage, muscle, blood, sperm, liver, kidney, lung, bone, hair, saliva, lymph, brain, CNS, PNS, breast, blood, blood vessel (e.g., artery or vein), fat, pancreas, liver, , gastrointestinal tract, heart, bladder, kidney, urethra, and prostate gland. In some options of the disclosure, cells such as primary immune cells, such as but not limited to T-cells, may be obtained from a biological sample, such as a blood sample obtained from a subject. In some options, a cell is genetically modified or engineered, and in some options an engineered cell is transgenic or edited.

Cells useful in options of methods of the disclosure may be maintained in cell culture following their isolation. Cells may be genetically modified or not genetically modified in various options of the disclosure. Cells may be obtained from normal or diseased tissue. In some options, cells are obtained from a donor, and their state or type is modified ex vivo using a method of the disclosure. In certain options of the disclosure a cell may be a free cell in culture, a free cell obtained from a subject, a cell obtained in a solid biopsy from a subject, organ, or solid culture, etc.

A population or plurality of isolated cells in any embodiment of the disclosure may be composed mainly or essentially entirely of a particular cell type or of cells in a particular state. In some options, an isolated population or plurality of cells consists of at least 30%, 40%, 50%, 60%, 70%, 80%, 90%, 95%, 96%, 97%, 98%, 99%, or 100% cells of a particular type or state (i.e., the population is at least 30%, 40%, 50%, 60%, 70%, 80%, 90%, 95%, 96%, 97%, 98%, 99%, or 100% pure), e.g., as determined by expression of one or more markers or any other suitable method.

In some options, a method of the disclosure is carried out on mammalian cells, including but not limited to cells from cell lines, cells obtained directly from a subject, primary immune cells (e.g., T-cells), cultured mammalian cells, transgenic mammalian cells, stem cells, diseased cells, and healthy cells. In some options, cells may be obtained from a living animal, such as a mammal or a non-mammal, or may be obtained from a collection of isolated cells. An isolated cell may be a primary cell, such as those recently isolated from an animal (e.g., cells that have undergone none or only a few population doublings and/or passages following isolation), or may be cells of a cell line that is capable of prolonged proliferation in culture (e.g., for longer than 3 months) or indefinite proliferation in culture (immortalized cells). In some options of the disclosure, a cell is a somatic cell. Somatic cells may be obtained from an individual, e.g., a human, and cultured according to standard cell culture protocols known to those of ordinary skill in the art.

In some options, a cell used in conjunction with the disclosure is a healthy normal cell, which is not known to have a disease, disorder, or abnormal condition. In some options, a cell used in conjunction with methods of the disclosure is an abnormal cell, for example, a cell obtained from a subject diagnosed as having a disorder, disease, or condition, including, but not limited to a degenerative cell, a neurological disease-bearing cell, a cell model of a disease or condition, an injured cell, etc. In some options of the disclosure, a cell is an abnormal cell obtained from cell culture, a cell line known to include a disorder, disease, or condition. In some options of the disclosure, a cell is a control cell. In some aspects of the disclosure a cell can be a model cell for a disease or condition.

### Additional Applications

Certain options of methods and systems of the disclosure can be applied to address biological s questions. For example, though not intended to be limited, certain options of methods and/or systems of the disclosure can be used to assess efficacy of a means of introducing a candidate genetic modification in a cell. Such an application may include assessing in a cell treated to introduce a candidate genetic modification in the cell, the sequences of the ligated cleaved fragments determined using a method of the disclosure described herein and identifying the presence or absence of the candidate genetic modification in the assessed determined sequences, wherein the presence of the candidate genetic modification confirms the efficacy of the means of introducing the candidate genetic modification in the cell. Some aspects of the disclosure include a system for performing the assessment of efficacy of a means of introducing a candidate genetic modification in a cell.

In another non-limiting example, certain options of methods and/or systems of the disclosure can be used to assess genetic variation in a cell. Such options of methods and/or systems of the disclosure may include obtaining genetic and epigenetic information in a genomic region containing a DNA sequence of interest; comparing the determined sequences of the ligated cleaved fragments (determined using a method of the disclosure described herein) to one or more reference sequences and identifying presence or absence of one or more differences between the determined ligated cleaved fragment sequences and the reference sequences, wherein the presence of one or more difference(s) indicate a genetic variation in the cell. In some options, the cell from which the ultralong DNA is extracted is a cell that is known to have or is suspected of having a disease or condition. In certain options, the reference sequence is the DNA sequence of interest extracted from a cell that does not have the disease or condition. In some options, the method and/or system also includes further assessment of the identified genetic variation and its effect or role in the disease or condition.

In another non-limiting example, certain options of methods and/or systems of the disclosure can be used to assess integration of an administered genetic material in a cell. In certain options such as method or system may include determining in the cell, using a method of the disclosure described herein, sequences of the ligated cleaved fragments of the DNA sequence of interest, wherein the cell comprises the administered genetic material and the administered genetic material comprises the DNA sequence of interest; comparing the sequences of the ligated cleaved fragments determined using a method of the disclosure described herein to one or more reference sequences; and identifying based on the comparing, whether the administered genetic material is one or more of: not integrated in the cell, integrated episomally in the cell, and integrated into the genome of the cell. It will be understood that the genetic material may be administered to the cell using standard means, which may include administering the genetic material to the cell in a vector. In some options, a vector used to administer the genetic material to the cell is an adeno-associated virus (AAV) vector, although other art-known vectors may be used. In some options the vector is a gene therapy vector and the administered genetic material includes therapeutic genetic material.

### Subjects

As used herein, the term "subject" may refer to a vertebrate or invertebrate animal (including humans), or a bacteria, or a fungus or a plant. In some options of the disclosure, a subject is a mammal, and in certain options, a subject is a human. In some options, a subject is a rodent, including but not limited to a mouse, rat, or hamster. In some options, a subject is a non-human primate, pig, fish, fruit fly, or other suitable vertebrate or invertebrate organism. In some options of methods of the disclosure, a subject is a subject that has been administered a therapy prior to assessment using a method of the disclosure. As a non-limiting example, a subject has been administered one or more gene therapy prior to assessment using a method of the disclosure. In some options of the disclosure, a subject is a normal, healthy subject and in some options, a subject is known to have, at risk of having, or suspected of having a disease or condition. In certain options of the disclosure, a subject is an animal model for a disease or condition. For example though not intended to be limiting, in some options of the disclosure a subject is a mouse that is an animal model for a disease or condition.

In some options of the disclosure, a subject is a wild-type subject. As used herein the term "wild-type" means the phenotype and/or genotype of the typical form of a species as it occurs in nature. In certain options of the disclosure a subject is a non-wild-type subject, for example, a subject with one or more genetic modifications compared to the wild-type genotype and/or phenotype of the subject's species. In some instances, a genotypic/phenotypic difference of a subject compared to wild-type results from a hereditary (germline) mutation or a somatic mutation. Factors that may result in a subject exhibiting one or more somatic mutations include but are not limited to: environmental factors, toxins, ultraviolet radiation, a spontaneous error arising in cell division, or a teratogenic event such as but not limited to radiation, maternal infection, or chemicals.

In certain options of methods of the disclosure, a subject is a genetically modified subject, also referred to as an engineered subject. An engineered subject may include one or more intentionally introduced and/or pre-selected genetic modifications, and may exhibit or be induced to exhibit one or more genotypic and/or phenotypic traits that differ from the traits in a non-engineered subject. In options, an engineered subject is transgenic, in which a transgene comprising one or more exogenous nucleic acid sequences has been inserted into its genome. Inserted nucleic acid sequences may be from the same species as the subject or from a different species. In some options, a genetically modified subject is edited, meaning that changes have been introduced into its genome by means of nuclease enzyme systems including but not limited to CRISPR-Cas9, CRISPR-Cas12, or TALEN. In certain options of the disclosure, routine genetic engineering techniques can be used to produce an engineered subject. A non-limiting example is a transgenic mouse in which a transgene encoding a promoter and optionally a fluorescent protein, such as green fluorescent protein (GFP), is inserted into the mouse's genome.

### Certain Options of Devices, Systems, and Methods

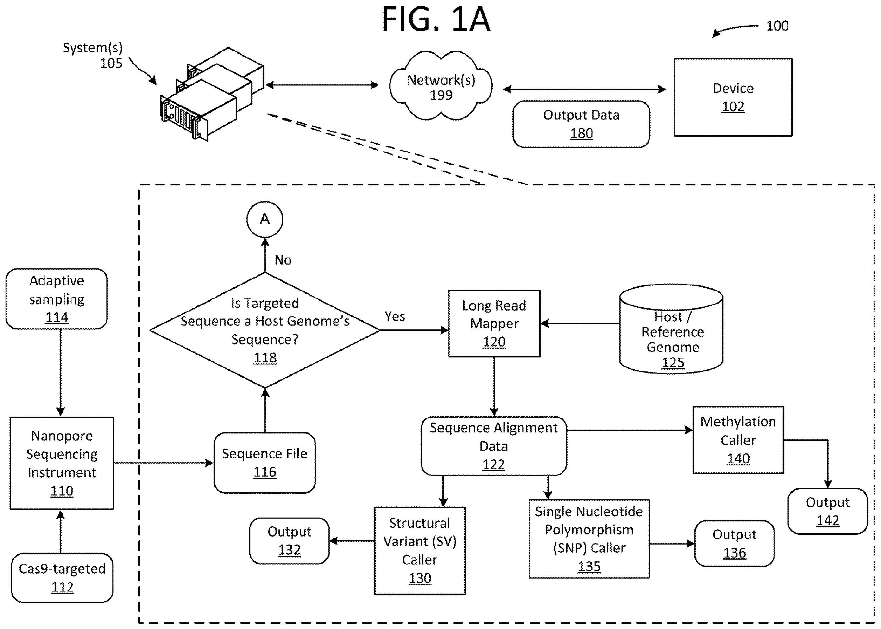

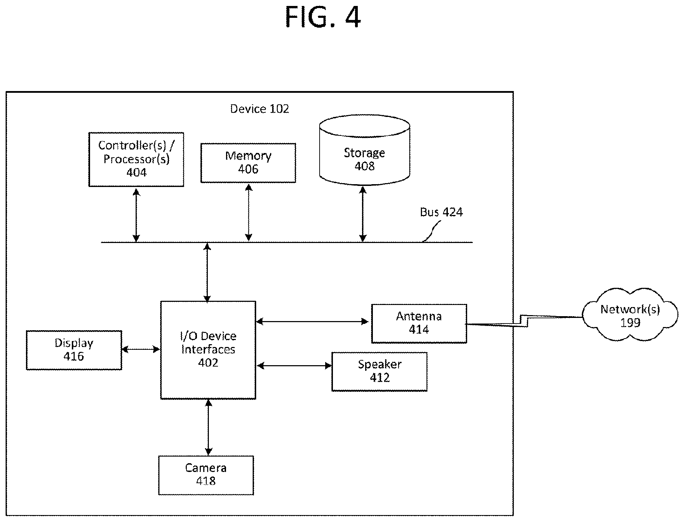

Some options of the present disclosure relate to a bioinformatics system, including one or more devices, systems and/or methods, for analyzing long-read sequencing data. FIGS. 1A-1B illustrate a system 100 configured to process data generated by different targeting sequencing strategies like Cas9-targeted, in-silico adaptive sampling, or other strategies. The system 100 may operate using various components as illustrated in FIGS. 1A-1B. The system 100 may include one or more systems 105 and a device 102 connected across one or more networks 199. The system(s) 105 may be a server(s), a computing system(s), networked computing devices/systems, etc. The device 102 may be a laptop, a desktop, a tablet, a smartphone, or other types of computing devices capable of displaying data (e.g., output data 180), receiving inputs from a user (e.g., a person wanting to analyze gene sequences), and may include one or more components described in connection with FIG. 4 below. The network(s) 199 may include the Internet and/or any other wide- or localarea network, and may include wired, wireless, and/or cellular network hardware.

The system 100 may involve use of multiple different / separate components to perform the functionalities described herein. One or more of these components may be included in the system(s) 105. In some options, one or more of these components may be implemented outside of the system(s) 105, and the system(s) 105 may invoke the component to perform its processing using, for example, an API call or by sending a request, command, instruction or the like, to the component, and the system(s) 105 may receive data from the component based on its processing. In other options, some of the components may be included / invoked by the system(s) 105, while other of the components may be included / invoked by the device 102. The components may include one or more software programs / instructions and/or one or more hardware components (e.g., for processing physical samples including DNA, gene, genomes, etc.).

The system 100 may also involve use of one or more data storages (e.g., database(s), data center(s), etc.) for storing sequencing data. The data storage(s) may be included in the system(s) 105, or may be in communication with the system(s) 105 / the device 102 over the network(s) 199. Some options may use a data center or data station that may be a supercomputer including one or more GPUs.

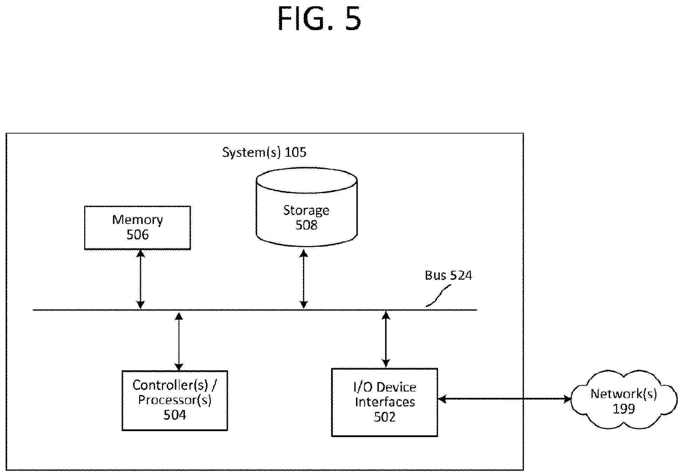

In one example embodiment, one or more CRISPR-Cas9 targeted cleavage 112 may be used for the isolation of a host's genome region of interest and then sequenced using a nanopore sequencing instrument 110. The nanopore sequencing instrument 110 may be configured to perform one or more nanopore sequencing techniques, which may be used in the sequencing of biopolymers, such as polynucleotides in the form of DNA or RNA. Using nanopore sequencing, a single molecule of DNA or RNA can be sequenced without the need for PCR amplification or chemical labeling of the sample. The nanopore sequencing instrument 110 may be an instrument / products provided by Oxford Nanopore Technologies, such as, MinION^{®}, PromethION^{®}, GridION, etc. In some options, the nanopore sequencing instrument 110 may include a physical device(s) configured to process physical samples of genes, a software program(s) to generate a sequence of the genes included in the physical sample, and a data storage for one or more files including the generated sequences. The nanopore sequencing instrument 110 may include (or may be in communication with) a device (e.g., a device with components shown in FIG. 4) or a system (e.g., a system with components shown in FIG. 5). The nanopore sequencing instrument 110 may be in communication with the system(s) 105 and/or the device 102 via the network(s) 199.

In another example embodiment, adaptive sampling 114 may be sequenced using the nanopore sequencing instrument 110. In this embodiment, the enrichment may be performed in real-time while sequencing, so that only DNA fragments from the genomic region of interest are sequenced - this process being controlled by the Nanapore Sequencing Instrument 110. As will be understood, adaptive sampling is a real-time software-controlled enrichment method that enables user to obtain a defined sequence selection from a whole genome library preparation, without the need for upfront sample preparation. The enrichment of the sequence of interest is performed by selecting molecules in real time during the sequencing process based on sequence identity [see for example: Payne, A., et al., Nat Biotechnol 39, 442-450 (2021) and Kovaka, S., et al., Nat Biotechnol 39, 431-441 (2021)].

Based on sequencing the input samples 112 or 114, the nanopore sequencing instrument may generate a sequence file 116. The sequence file 116 may include one or more sequences, and may be of one or more of following file formats: FAST5, FASTA, FASTQ, or other sequence file formats. In some options, the system(s) 105 may process the sequence file generated by the nanopore sequencing instrument 110 to convert it to another file format (a desired file format) for the sequence file 116. For example, the nanopore sequencing instrument 110 may output a sequence file in a FAST5 format, and the system(s) 105 may convert that to the sequence file 116 having a FASTQ file format. The system(s) 105 may use one or more techniques to convert the file formats, such as, basecalling algorithms / software, which may involve barcoding / demultiplexing, adapter trimming and alignment, modified basecalling (5mC, 6mA and CpG) from the raw signal data, producing an additional FAST5 file of modified base probabilities, etc.

The FAST5 file format may be a standard sequencing output for Oxford Nanopore sequencers such as the MinION^{®}, and is based on a hierarchical data format (HDF5 format) which enables storage of large and complex data. In contrast to FASTA and FASTQ files, a FAST5 file is binary and may not be opened with a generic text editor. Data stored in FAST5 files can contain the sequence of a read in FASTQ format (after basecalling), the raw signal of the pore as well as several log files (based on processing by the nanopore sequence instrument 110) and other information.

The FASTA format is one of the simplest and common file formats to store sequence data. A FASTA file can contain one or many nucleotide or amino acid sequences. The first line of a sequence in a FASTA file may start with a ">" followed by a series of identifiers or attributes. Subsequent lines contain the nucleotide or amino acid sequence.

The FASTQ format may be the standard file of certain generation sequencing technologies, and may be similar to the FASTA format but in addition to the sequence itself a FASTQ file also stores quality scores of the sequence. A FASTQ file may store every sequence in four lines: (1) the name/ID line starting with "@" followed by a identifier; (2) the sequence itself; (3) a line starting with "+" (optionally followed by additional information, e.g., the read names again); (4) the quality line with one character per sequence residue encoding the probability of a possible sequencing error (e.g., Phred score).

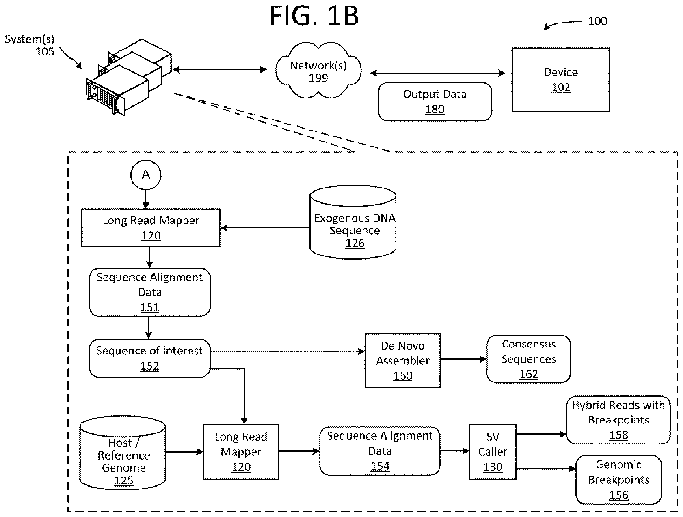

The system(s) 105 may determine, at a decision block 118, whether the targeted sequence is a host genome's sequence. In some options, the system(s) 105 may receive an input indicating whether the targeted sequence is a host genome sequence or an exogenous DNA sequence. Such input (e.g., keyboard input, mouse click input, etc.), in some options, may be provided by a user using the device 102 via a user interface. If the targeted sequence is not a host genome's sequence, then the system(s) 105 may perform the steps shown in FIG. 1B. If the targeted sequence is a host genome's sequence, then the system(s) 105 may process the sequence file 116 using a long read mapper component 120. The long read mapper component 120 may align the sequences in the sequence file 116 to reference sequences stored in a host / reference genome storage 125. The host / reference genome storage 125 may store sequences of a single host / reference genome or multiple hosts / reference genomes each associated with an identifier of the host (e.g., an alphanumerical identifier, a host name, etc.). The long read mapper 120 may output sequence alignment data 122 identifying sequences from the sequence file 116 that align with the host / reference genome in storage 125.

In example options, the long read mapper component 120 may be implement a CoNvex Gap-cost align Ments for Long Reads (NGMLR) mapper. The NGMLR mapper may be designed to quickly and correctly align the reads of interest, including those spanning complex structural variants (SVs). The NGMLR mapper may use the convex gap-cost scoring model to accurately align long reads across small indels that commonly occur as sequencing errors. Larger and complex SVs may be captured through spot-read alignments.

In other options, the long read mapper component 120 may implement a Minimap2 aligner. The Minimap2 aligner may be a whole genome aligner using, for example, a seedchain-align procedure. The Minimap2 aligner may index the minimizers of the host / reference genome and store a list of locations of the minimizer copies as a value. Then, Minimap2 aligner may take query minimizers and finds exact matches to the reference / host genome for each query sequence in the sequence file 116. A set of collinear matches to the reference / host genome may be identified as chains. The Minimap2 aligner may then perform a dynamic programming-based global alignment between adjacent matches to the reference / host genome in a chain.

The system(s) 105 may process the sequence alignment data 122 using a structural variant (SV) caller component 130, which may generate an output 132. The SV caller component 130 may be configure to perform one or more structural variant (SV) calling techniques. SV are genomic alterations that may involve DNA segments larger than 1 kilobase (kb). Examples of SVs include insertions, deletions, inversions, duplications, translocations, copy-number variants (CNVs) and the like. In an example embodiment, the SV caller component 130 may implement a Structural Variant Identification Method (SVIM), which may be a SV caller that can be used for large nested structural variants. The SVIM technique may detect deletions, insertions, tandem and interspersed duplications, inversions and novel element insertions. The SVIM technique may consist of three components: collection, clustering and combination of structural variant signatures from read alignments. The SV caller component 130 can implement other SV calling techniques in other options. The output 132 may be the sequence file 116 that represents structural variants in the sequence of interest regarding the host / reference genome and / or a file containing the coordinates (e.g., genomic positions) where the breakpoints of the structural variants were identified, for example, in BED format.

The system(s) 105 may process the sequence alignment data 122 using a single nucleotide polymorphism (SNP) caller component 135, which may generate an output 136. The SNP caller component 135 may be configured to perform one or more single nucleotide polymorphism (SNP) calling techniques. The SNP caller component 135 may be configured to determine in which positions / portion, of the sequence file 116, there are polymorphisms or in which positions / portions, of the sequence file 116, at least one of the bases differs from the reference / host sequence, based on processing the sequence alignment data 122. The SNP caller component 135 may also involve using a probabilistic framework. The output 136 may be a Variant Calling Format (VCF) file containing information regarding each variable position (e.g., genomic coordinates, sequences supporting the SNP, etc.)

In an example embodiment, the SNP caller component 135 may implement the Medaka tool (provided by Oxford Nanopore Technologies), which may create consensus sequences and variant calls from nanopore sequencing data included in the sequence alignment data 122. The Medaka tool may use one or more machine learning models, such as, neural networks, to apply a pileup of individual sequencing reads against a draft assembly or reference sequence. In another example embodiment, the SNP caller component 135 may use a graph-based method, which may operate on basecalled data in some options. The SNP caller component 135 can implement other SNP calling techniques in other options.

The system(s) 105 may also process the sequence alignment data 122 using a methylation caller component 140, which may generate an output 142. The methylation caller component 140 may be configured to perform one or more methylation calling techniques. The methylation caller component 140 may be configured to identify DNA modifications, which play a fundamental role in genome stability and gene regulation during subject development, disease progression, and aging. In some options, the methylation caller component 140 may be configured to identify the methylation of cytosines at CG dinucleotides (CpG), involving the addition of a methyl group (-CH3) to the 5th carbon of the cytosine ring to form 5-methylcytosine (5mC), is the most frequently observed methylation in relation to gene regulation. The methylation caller component 140 may implement one or more machine learning models, probabilistic models, and/or statistical models to perform methylation. The output 142 may include the position of the CG dinucleotide on the reference genome and the frequency (e.g., value ranging from 0 to 1) indicating the portion of the sequence file 116 containing methylation in each position.

In an example embodiment, the methylation caller component 140 may implement a Nanopolish tool (provided by Oxford Nanopore Technologies), which may perform signallevel analysis to detect base modifications. The Nanopolish tool may detect CpG methylation using a hidden Markov model.

Referring to FIG. 1B, the system(s) 105 may perform the steps / processing shown in FIG. 1B if the targeted sequence is not a host genome's sequence (as determined at the decision block 118 shown in FIG. 1A). The system(s) 105 may process the sequence file 116, using the long read mapper 120 (shown in FIG. 1A, or another instance or type of long read mapper) , and data stored at an exogenous DNA sequence storage 126, to create sequence alignment data 151. The sequence alignment data 151 may be analyzed to determine an alignment between the sequences included in the sequence file 116 and the sequences included in the exogenous DNA sequence storage 126. The exogenous DNA sequence storage 126 may store sequences for one or more viruses, one or more vectors (e.g., an Ad vector, a transgene, a regulatory cassette, etc.), or other non-naturally occurring / nonhost sequences. The sequence alignment data 151 may be a BAM file format. A BAM file is a binary version of a tab-delimited text file (e.g., a SAM file format) that contains sequence alignment data.

In an example embodiment, the long read mapper 120 may implement the Minimap2 aligner described above. The long read mapper 120 component 120 can implement other alignment techniques in other options.

The sequence alignment data 151 may identify reads, from the sequence file 116, that include a sequence of interest, where the sequence of interest may be an exogenous sequence, and may output those reads as a sequence of interest 152. The system(s) 105 may process the sequence alignment data 151 (that may be in the BAM file format) to extract sequences mapping to the sequence of interest 152. In some options, the sequence of interest 152 may be a FASTQ file format.

The system(s) 105 may generate the sequence alignment data 151 using the long read mapper component 120 (described above in relation to FIG. 1A). The long read mapper component 120 may map reads containing the sequence of interest 152 to the host / reference sequences retrieved from the host / reference genome storage 125. Sequence alignment data 154 outputted by the long read mapper 120 may indicate an alignment / mapping between the exogenous DNA and the host / reference genome. In some options, the sequence alignment data 154 may be BAM file. In other options, the sequence alignment data 154 may be another file format.

The system(s) 105 may then process the sequence alignment data 154 using the SV caller component 130 (described above in relation to FIG. 1A). In an example embodiment, the SV caller component 130 may use the SVIM technique (described above in relation to FIG. 1A) to process the sequence alignment data 154. The SV caller component 130 may be configured to determine genomic breakpoints 156 based on the alignment, indicated in the sequence alignment data 154, between the host / reference genome and the exogenous DNA. In an example embodiment, the genomic breakpoints 156 may be included in a Browser Extensible Data (BED) file format. A BED file is a text file format used to store genomic regions, as coordinates, along with associated annotations. The data in the BED file may be presented in the form of columns separated by spaces or tabs. In other options, the genomic breakpoints 156 may be included in another type of file format.

The SV caller component 130 may also (or instead) be configured to determine hybrid reads with breakpoints 158 based on the alignment, indicated in the sequence alignment data 154, between the host / reference genome and the exogenous DNA. In an example embodiment, the hybrid reads with breakpoints 158 may be included in a FASTQ file. In other options, the hybrid reads with breakpoints 158 may be included in another type of file format.

The system(s) 105 may process reads containing the sequence of interest 152 using a de novo assembler component 160. The de novo assembler component 160 may be configured to assemble short nucleotide sequences into longer ones without the use of a reference genome. The de novo assembler component 160 may use one or more de novo assemblers techniques. Sequence reads may be assembled as contigs, and the coverage quality of the de novo sequence data may depend on the size and continuity of the contigs (i.e. the number of gaps in the data). De novo techniques may involve one or more graphtheory models, probabilistic models, statistical models, machine learning models, etc., configured to exploit overlap information to stitch together the short reads into contiguous sequences. One example de novo assembler may use a greedy algorithm. Another example de novo assembler may use a De Bruijn graph technique. The de novo assembler component 160 may output one or more consensus sequences 162 representative of the sequence of interest in the subject.

In an example embodiment, the de novo assembler component 160 may implement a Flye tool, which may be a de novo assembler for single molecule sequencing reads. The Flye tool may be used for a wide range of datasets.

After or while performing the above described steps / processing of FIGS. 1A-1B, the system(s) 105 may send the output data 180 to the device 102 for a user to view. The output data 180 may include one or more of: sequence file 116, the sequence alignment data 122, the output 132, the output 136, the output 142, the reads containing the sequence of interest 152, the sequence alignment data 154, the genomic breakpoints 156, the hybrid reads with breakpoints 158, and the consensus sequences 162.

Although FIGS. 1A and 1B show certain steps and processing occurring in a particular order, it should be understood that the steps and processing may occur in a different order and/or in parallel to one another. For example, the SV caller component 130, the SNP caller component 135 and the methylation caller component 140 may process in parallel / at substantially the same time. As another example, the long read mapper 120 and the de novo assembler component 160 may process in parallel / at substantially the same time.

In this manner, the system 100 provides an optional parameter for the analysis of (at least) two kind of targeted sequencing experiments depending on the origin of the targeted sequenced: 1) targeted of a specific region of a known genome (reference sequence) or 2) targeted exogenous DNA inserted in a known genome (reference sequence).

In the case of targeting a region of the reference genome (as described in relation to FIG. 1A above), the system 100, in some options, first aligns ONT reads (e.g., the sequence file 116) to the reference genome using Minimap2 or NGMLR (e.g., the long read mapper 120), and then calls structural variants using SVIM (e.g., the SV caller component 130) and small INDELS using Medaka (e.g., the SNP caller component 135) in the region of interest. The system 100 also allows performing DNA modification analysis (5mc methylation) on the region of interests using Nanopolish (e.g., the methylation caller component 140).

In the case of exogenous DNA inserted in a known genome (as described in relation to FIG. 1B above), the system 100 can identify any integration event, such as those produced by genome editing experiments or, for instance, viral genome integrations. To detect integration events or any other non-reference insertions, the system 100, in some options, first uses Minimap2 (e.g., the alignment component 150) to map ONT / FASTQ reads from the query samples to indexed set of sequences of interest (e.g. Ad vectors, transgene, regulatory cassette, etc.). Reads containing the sequence of interest 152 are then mapped by the system 100 to the reference / host genome using NGMLR (e.g., the long read mapper 120). Then, SVIM (e.g., the SV caller component 130) is applied to identify reads with insertions embedded in the host's genome1 and genomic breakpoints (e.g., 156), which in case on integration event experiments corresponds to the genomic sites of vector integration. Reads that align with the exogenous DNA sequence 126 and the host genome, and show evidence of insertions according to SVIM are classified as 'hybrid' reads (e.g., 158). Reads aligning to the exogenous DNA sequence 126 are also used to reconstruct the inserted sequences (e.g., 162) using a de novo assembly approach (e.g., the de novo assembler component 160), which allows identification of both in-tandem integration events as well as episomal sequences.

The system 100 provides an all-in-one solution for the processing, analysis and interpretation of long-read targeted sequencing data produced using different sequencing strategies and setups. For experiments targeting a specific region of a known genome, the system 100 can report: basic QC of the sequenced reads (Phred scores distribution, read length distribution, N50, etc., which may be determined from the sequence file 116), the target and off-target report (efficiency, which may be derived from the sequence alignment data 122), structural variation report (e.g., outputted by the SV caller component 130 shown in FIG. 1A), SNPs and INDELs report (e.g., outputted by the SNP caller component 135), methylation calls (e.g., outputted by the methylation caller component 140), consensus sequence (e.g., outputted by the SNP caller 135), and putatively affected genes or regulatory regions.

In the case of exogenous DNA inserted in a known genome (e.g. vector integration assays, genome-editing experiments, etc.), the system 100 can report: basic QC of the sequenced reads (Phred scores distribution, read length distribution, N50, etc., which may be determined from the sequence file 116), number of integration/insertion events and their genomic coordinates (accompanied by a genome browser picture; e.g., outputted by the SV caller component 130 shown in FIG. 1B), number of reads supporting the integration event (e.g., from the hybrid reads with breakpoints 158), putatively affected genes or regulatory regions (e.g., from the genomic breakpoints 156), consensus sequence of the integrated and/or the episomal sequences (e.g., outputted by the de novo assembler component 160).

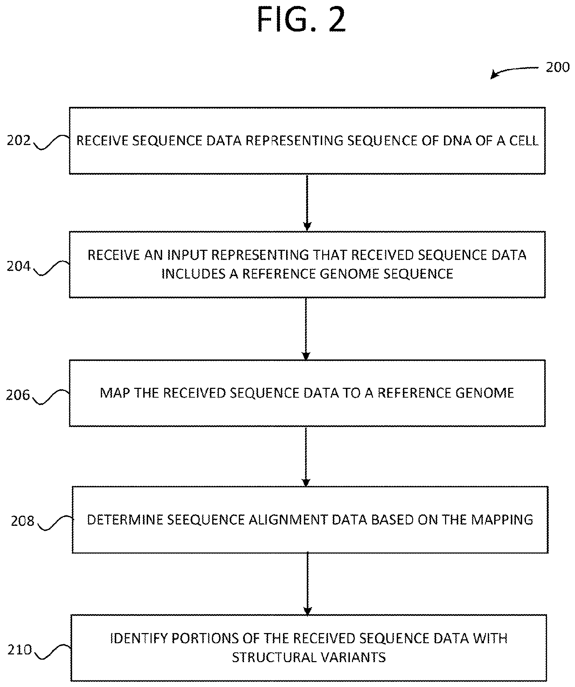

FIG. 2 is a flowchart illustrating a process 200 for characterizing a genomic region expected to be, at least, partially present in the reference genome of a cell, according to options of the present disclosure. One or more of the steps 200 may be performed in another order / sequence than shown in FIG. 2. One or more steps of the process 200 may be performed by the components of the system(s) 105 illustrated in FIG. 1A.

At a step 202, the system(s) 105 may receive sequence data representing sequences of DNA of the cell, where the sequences may be determined using a nanopore sequencing technique (e.g. the nanopore sequence instrument 110). At a step 204, the system(s) 105 may receive an input representing that the received sequence data includes a reference genome sequence. In response determining that the received sequence data includes the reference genome sequence, based on the received input, the system(s) 105 may, at a step 206, map the received sequence data to a reference genome using a long read mapper technique (e.g., the long read mapper 120). At a step 208, the system(s) 105 may determine sequence alignment data based on the mapping of the received sequence data to the reference genome, and at a step 210, the system(s) 105) may identify portions of the received sequence data with structural variants based on the sequence alignment data. Such identifying (of step 210) may be performed using a structural variant identification technique (e.g., the SV caller component 130 and/or the SNP caller component 135). Additionally, in some options, the system(s) 105 may identify single nucleotide polymorphisms within the sequence alignment data. Additionally, in some options, the system(s) 105 may identify a DNA modification within the sequence alignment data, where the identifying may be performed using a methylation technique (e.g., the methylation caller component 140).

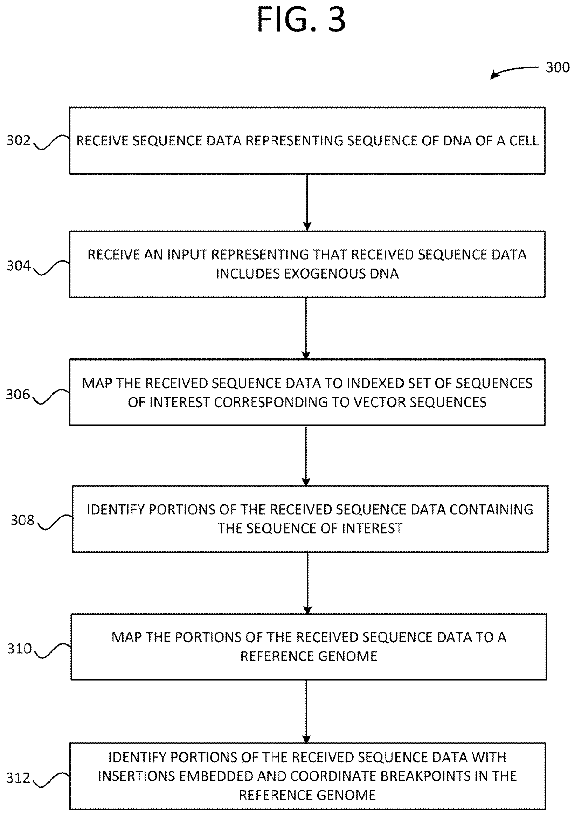

FIG. 3 is a flowchart illustrating a process 300 for identifying integration events of an exogenous DNA within a cell, according to options of the present disclosure. One or more of the steps 300 may be performed in another order / sequence than shown in FIG. 3. One or more steps of the process 300 may be performed by the components of the system(s) 105 illustrated in FIG. 1B.

At a step 302, the system(s) 105 may receive sequence data representing sequences of DNA of the cell, the sequences being determined using a nanopore sequencing technique (e.g. the nanopore sequence instrument 110). At a step 304, the system(s) 105 may receive an input representing that the received sequence data includes exogenous DNA. In response to receiving the input representing that the received sequence data includes exogenous DNA, the system(s) 105, may, at a step 306, map (e.g., using the alignment component 150) the received sequence data to an indexed set of DNA sequences of interest, wherein the indexed set may comprise sequences of at least one vector (e.g., an Ad vector, a transgene, a regulatory cassette, etc.). At a step 308, the system(s) 105 may identify one or more portions of the received sequence data containing the DNA sequence of interest, based on the mapping performed in step 306. At a step 310, the system(s) 105 may map the portions of the received sequence data containing the DNA sequence of interest to a reference genome using a long read mapper technique (e.g., the long read mapper 120). At a step 312, the system(s) 105 may identify portions of the received sequence data with insertions embedded and the coordinate breakpoints in the reference genome, where the identifying may be performed using a structural variant identification technique (e.g., the SV caller component 130) and using the mapping (performed in step 310) of the portions of the received sequence data containing the DNA sequence of interest to the reference genome. Additionally, the system(s) 105 may identify the portions of the received sequence data as hybrid sequences based on the portions aligning with the indexed set of DNA sequences of interest and the reference genome. Additionally, the system(s) 105 may reconstruct, using a de novo assembler (e.g., the de novo assembler component 160), one or more inserted sequences from the portions of the received sequence data. Additionally, the system(s) 105 may identify, based on the reconstructed inserted sequence(s), in-tandem integration events within the cell. Additionally, the system(s) 150 may identify, based on the reconstructed inserted sequence(s), episomal sequences within the cell.

FIG. 4 is a block diagram conceptually illustrating a device 102 that may be used with the system. FIG. 5 is a block diagram conceptually illustrating example components of a remote device, such as the system(s) 105, which may facilitate processing of DNA sequences, etc. A system(s) 105 may include one or more servers. A "server" as used herein may refer to a traditional server as understood in a server / client computing structure but may also refer to a number of different computing components that may assist with the operations discussed herein. For example, a server may include one or more physical computing components (such as a rack server) that are connected to other devices / components either physically and/or over a network and is capable of performing computing operations. A server may also include one or more virtual machines that emulates a computer system and is run on one or across multiple devices. A server may also include other combinations of hardware, software, firmware, or the like to perform operations discussed herein. The server(s) may be configured to operate using one or more of a clientserver model, a computer bureau model, grid computing techniques, fog computing techniques, mainframe techniques, utility computing techniques, a peer-to-peer model, sandbox techniques, or other computing techniques.

Multiple systems 105 may be included in the overall system of the present disclosure, such as one or more systems 105 for determining sequence alignment data, one or more systems 105 for determining structural variants, one or more systems 105 for determining DNA modifications, one or more systems 105 for determining genomic breakpoints, one or more systems 105 for determining hybrid reads, etc. In operation, each of these systems may include computer-readable and computer-executable instructions that reside on the respective device / system 105, as will be discussed further below.

Each of these devices (102/105) may include one or more controllers/processors (404/504), which may each include a central processing unit (CPU) for processing data and computer-readable instructions, and a memory (406/506) for storing data and instructions of the respective device. The memories (406/506) may individually include volatile random access memory (RAM), non-volatile read only memory (ROM), non-volatile magnetoresistive memory (MRAM), and/or other types of memory. Each device (102/105) may also include a data storage component (408/508) for storing data and controller/processor-executable instructions. Each data storage component (408/508) may individually include one or more non-volatile storage types such as magnetic storage, optical storage, solid-state storage, etc. Each device (102/105) may also be connected to removable or external non-volatile memory and/or storage (such as a removable memory card, memory key drive, networked storage, etc.) through respective input/output device interfaces (402/502).

Computer instructions for operating each device (102/105) and its various components may be executed by the respective device's controller(s)/processor(s) (404/504), using the memory (406/506) as temporary "working" storage at runtime. A device's computer instructions may be stored in a non-transitory manner in non-volatile memory (406/506), storage (408/508), or an external device(s). Alternatively, some or all of the executable instructions may be embedded in hardware or firmware on the respective device in addition to or instead of software.

Each device (102/105) includes input/output device interfaces (402/502). A variety of components may be connected through the input/output device interfaces (402/502), as will be discussed further below. Additionally, each device (102/105) may include an address/data bus (424/524) for conveying data among components of the respective device. Each component within a device (102/105) may also be directly connected to other components in addition to (or instead of) being connected to other components across the bus (424/524).

Referring to FIG. 4, the device 102 may include input/output device interfaces 402 that connect to a variety of components such as an audio output component such as a speaker 412, a wired headset or a wireless headset (not illustrated), or other component capable of outputting audio. The device 102 may additionally include a display 416 for displaying content. The device 102 may further include a camera 418.

Via antenna(s) 414, the input/output device interfaces 402 may connect to one or more networks 199 via a wireless local area network (WLAN) (such as WiFi) radio, Bluetooth, and/or wireless network radio, such as a radio capable of communication with a wireless communication network such as a Long Term Evolution (LTE) network, WiMAX network, 3G network, 4G network, 5G network, etc. A wired connection such as Ethernet may also be supported. Through the network(s) 199, the system may be distributed across a networked environment. The I/O device interface (402/502) may also include communication components that allow data to be exchanged between devices such as different physical servers in a collection of servers or other components.

The components of the device(s) 102 or the system(s) 105 may include their own dedicated processors, memory, and/or storage. Alternatively, one or more of the components of the device(s) 102, or the system(s) 105 may utilize the I/O interfaces (402/502), processor(s) (404/504), memory (406/506), and/or storage (408/508) of the device(s) 102, or the system(s) 105, respectively.

As noted above, multiple devices may be employed in a single system. In such a multi-device system, each of the devices may include different components for performing different aspects of the system's processing. The multiple devices may include overlapping components. The components of the device 102, and the system(s) 105, as described herein, are illustrative, and may be located as a stand-alone device or may be included, in whole or in part, as a component of a larger device or system.

The concepts disclosed herein may be applied within a number of different devices and computer systems, including, for example, general-purpose computing systems, video / image processing systems, and distributed computing environments.

The above aspects of the present disclosure are meant to be illustrative. They were chosen to explain the principles and application of the disclosure and are not intended to be exhaustive or to limit the disclosure. Many modifications and variations of the disclosed aspects may be apparent to those of skill in the art. Persons having ordinary skill in the field of computers and speech processing should recognize that components and process steps described herein may be interchangeable with other components or steps, or combinations of components or steps, and still achieve the benefits and advantages of the present disclosure. Moreover, it should be apparent to one skilled in the art, that the disclosure may be practiced without some or all of the specific details and steps disclosed herein.

Aspects of the disclosed system may be implemented as a computer method or as an article of manufacture such as a memory device or non-transitory computer readable storage medium. The computer readable storage medium may be readable by a computer and may comprise instructions for causing a computer or other device to perform processes described in the present disclosure. The computer readable storage medium may be implemented by a volatile computer memory, non-volatile computer memory, hard drive, solid-state memory, flash drive, removable disk, and/or other media. In addition, components of system may be implemented as in firmware or hardware.

Various exemplary options of compositions and methods according to this disclosure are now described in the following non-limiting Examples. The Examples are offered for illustrative purposes only and are not intended to limit the scope of the present disclosure in any way. Indeed, various modifications of the disclosure in addition to those shown and described herein will become apparent to those skilled in the art from the foregoing description and the following examples and fall within the scope of the appended claims.

### EXAMPLES

### Example 1. Long-read Cas9 targeted sequencing workflow and analysis pipeline

Library preparation and sequence analysis strategies were developed for long-read Cas9 targeted sequencing.

### Materials and Methods

### Extraction Protocols for extraction from multiple sample types

High molecular weight (HMW) genomic DNA (gDNA) may be extracted from multiple sample types as described herein below.

### Tissue Extraction

For gDNA extraction, tissue was pulverized on dry ice in a Spectrum^{™} Bessman Tissue Pulverizer (ThermoFisher Scientific, Waltham, MA). HMW gDNA extraction was performed on tissues isolated from N1 or higher generation mice using a Monarch^{®} HMW DNA Extraction Kit for Tissue (New England BioLabs, Ipswich, MA) according to manufacturer's instructions. Low rpm lysed sample types were mixed by rotation at 10 rpm for 8 minutes (min) rather than 4 min, resulting in greater DNA binding to the beads. In certain studies fresh or frozen tissue from mice of any background was used.

### Cryopreserved sperm cells

HMW gDNA was extracted with minimal shearing from cryopreserved sperm cells using a gentle lysis and extraction protocol. Sperm cells were defrosted on ice and subsequently pelleted at 1000 x g for 3 min at 4°C. Cells were lysed at 56°C with shaking at 300-2000 rpm in SCL buffer (10 mM Tris pH 8, 150 mM NaCl, 1 mM EDTA, 1 mM DTT, 0.1% SDS, and 0.5% Tween^{®}-20) for 20-30 min. Use of detergent (Tween^{®} or Triton^{™} X-100) and reducing agents such as DTT was preferred in this protocol. Reducing agents and Proteinase K resulted in degradation of the protein shell surrounding the sperm gDNA with minimal damage. After initial lysis, the resulting homogenate was used in the Monarch^{®} HMW gDNA protocol described herein under Tissue Extraction.

### Cell extraction

Cell and blood extraction protocols were performed using a Monarch^{®} HMW DNA Extraction Kit for Tissue, according to manufacturer's instructions (New England BioLabs, Ipswich, MA).

### Cas9 Library preparation

### Cas9 ribonucleoprotein complex (RNP) preparation

Cas9 (Alt-R^{®} S.p. HiFi Cas9 nuclease, Integrated DNA Technologies, Coralville, IA) was loaded with crRNA (Alt-R^{®} S.p. Cas9 crRNA, resuspended at 100 µM in Tris base and EDTA (TE) pH 7.5; Integrated DNA Technologies, Coralville, IA) and tracrRNA (Alt-R^{®} S.p. Cas9 tracrRNA, resuspended at 100 µM in TE pH 7.5; Integrated DNA Technologies, Coralville, IA) to form RNPs in preparation for the cleavage reaction. The protocol described below herein describes RNP formation with a single crRNA.

A thermal cycler was pre-heated to 95°C, and an aliquot of Reaction Buffer (RB; Oxford Nanopore Technologies, Oxford, UK) was thawed, mixed by vortexing, and placed on ice. In a 0.2 ml PCR tube, crRNA probes for each cleavage reaction were pooled by combining equal volumes of each crRNA probe (resuspended at 100 µM in TE pH 7.5). For single-cut strategies, two separate cleavage reactions were prepared, each with one crRNA (5' or 3' crRNA), and resulting libraries were pooled at a later step. For a tiling approach, a single cleavage reaction may use one or more crRNA probes, up to 100 probes. Pooled crRNAs were annealed with tracrRNA in nuclease-free Duplex Buffer (Integrated DNA Technologies, Coralville, IA) by assembling the following reaction in a 0.2 ml thinwalled PCR tube.

**Table 1. crRNA*tracrRNA annealing mix**

| **Reagent** | **Volume (µl)** |
|---|---|
| Duplex Buffer | 8 |
| crRNA pool (100 µM, equimolar) | 1 |
| tracrRNA (100 µM) | 1 |
| **TOTAL** | **10** |

The annealing mix reaction was mixed by pipetting and spun down. The reaction was heated at 95°C for 5 min, allowed to cool to room temperature (RT) for 10 min, and spun down to collect any liquid. Storage and reuse of the annealed mix was not preferred.

To form Cas9 RNPs, the components in Table 2 were assembled in order in a 1.5 ml DNA LoBind (Eppendorf, Framingham, MA) tube:

**Table 2. Cas9 RNP reaction mix**

| **Number of Reactions** | **3** | **5** | **10** |
|---|---|---|---|
| **Reagent** | **Volume (µl)** | **Volume (µl)** | **Volume (µl)** |
| Annealed crRNA*tracrRNA mix (10 µM) | 3 | 5 | 10 |
| Reaction Buffer (RB) | 3 | 5 | 10 |
| Nuclease-free water | 23.7 | 39.6 | 79.2 |
| HiFi Cas9 (62 µM) | 0.3 | 0.4 | 0.8 |
| **Total** | **30 µl** | **50 µl** | **100 µl** |

The reaction was mixed thoroughly by flicking the tube. RNPs were formed by incubating the tube at RT for 30 min, then placing the tube on ice.

To form panels of multiple crRNAs, each crRNA was diluted, heated, and snap-cooled independently, in separate tubes, one crRNA per tube, and was bound to preformed Cas9-tracrRNA complex in a 1:1 ratio (v/v), according to the single crRNA protocol above. Once formed, individual Cas9-tracrRNA-crRNA ribonucleoprotein complexes (RNPs) were recombined into a single tube before being bound to target(s).

For each reaction, 10 µl of RNPs were carried forward into the next target cleavage step. Dephosphorylation of gDNA (described below herein) was performed during the 30 min RNP incubation.

### Dephosphorylation of gDNA

One to ten micrograms HMW gDNA in TE (pH 8.0) or nuclease-free water (5-10 µg in nuclease-free water was preferred) was transferred into a 0.2 ml thinwalled PCR tube, adjusted to 24 µl total volume with nuclease-free water, and was mixed thoroughly by flicking the tube to avoid unwanted shearing, and spun down briefly. The following components were assembled in a clean 1.5 ml DNA LoBind tube:

**Table 3. Dephosphorylation reaction mix**

| **Reagent** | **Volume (µl)** |
|---|---|
| Reaction Buffer (RB) | 3 |
| HMW gDNA (≥ 210 ng/µl) | 24 |
| **Total** | **27** |

The reaction was mixed gently by flicking the tube and was spun down. Phosphatase (PHOS; Oxford Nanopore Technologies, Oxford, UK) was mixed at RT by pipetting up and down; 3 µl of PHOS was added to the reaction tube, giving a total reaction volume of 30 µl; and the reaction tube was mixed gently by flicking the tube and was spun down. The reaction was incubated in a thermal cycler under the following conditions: 37°C (10 min), 80°C (2 min), then held at RT.

### Cleaving and dA-tailing target DNA

Cas9 RNPs and Taq polymerase (Oxford Nanopore Technologies, Oxford, UK) were added to the dephosphorylated HMW gDNA sample. This step cleaved the gDNA at target sites and dA-tailed all available DNA ends, activating the Cas9 cut site for ligation. The dATP tube (Oxford Nanopore Technologies, Oxford, UK) was thawed, vortexed to mix thoroughly, and placed on ice; the Taq polymerase tube was spun down and placed on ice. The following reagents were added to the tube containing the 30 µl dephosphorylated DNA sample:

**Table 4. Cleavage and dA-tailing reaction mix**

| **Reagent** | **Volume (µl)** |
|---|---|
| Dephosphorylated DNA | 30 |
| Cas9 RNPs | 10 |
| 10 mM dATP | 1 |
| Taq | 1 |
| **Total** | **42** |

The reaction was carefully mixed by gentle inversion, spun down, and was incubated in a thermal cycler under the following conditions: 37°C (30 min, Cas9 enzyme active), 72°C (5 min, Cas9 denatured). The completed reaction was held at 4°C or placed on ice. One of skill will understand that appropriate 37°C incubation times may vary according to the needs of the experiment, and that routine experimentation may include varying incubation times. Longer 37°C incubations may increase the amount of off-target reads without increasing the yield of on-target reads, while shorter incubations may result in incomplete target cleavage. However, some regions may benefit from a longer incubation at 37°C.

### Adapter Ligation

Adapters (Oxford Nanopore Technologies, Oxford, UK) were ligated to the free ends generated by Cas9 cleavage. Ligation Buffer (LNB; Oxford Nanopore Technologies, Oxford, UK) was thawed at RT, spun down, mixed thoroughly by pipetting, and placed on ice immediately after thawing and mixing. An aliquot of Adapter Mix (AMX; Oxford Nanopore Technologies, Oxford, UK) was thawed at RT, mixed by flicking the tube, pulse-spun, and placed on ice. The following adapter ligation mix was assembled at RT, with AMX last, added last and immediately before the ligation step:

**Table 5. Adapter ligation mix**

| **Reagent** | **Volume (µl)** |
|---|---|
| Ligation Buffer (LNB) | 20 |
| Nuclease-free water | 3 |
| T4 Ligase (LIG) | 10 |
| Adapter Mix (AMX)* | 5 |
| **Total** | **38** |

The adapter ligation mix was mixed by pipetting thoroughly. The cleaved and dA-tailed gDNA sample was transferred from the 0.2 ml PCR tube to a 1.5 ml LoBind (Eppendorf, Framingham, MA) tube. Half the volume (19 µl) of the adapter ligation mix was added to the cleaved and dA-tailed gDNA sample, and the ligation reaction was mixed by flicking the tube. Immediately after mixing, the remainder of the adapter ligation mix was added to the ligation reaction (80 µl final volume). The ligation reaction was mixed gently by flicking the tube and spun down. The reaction was incubated for 1 hour at RT, resulting in a greater number of DNA-adaptor complexes. Adding the adapter ligation mix in two parts helped reduce formation of a white precipitate that was sometimes observed upon addition of the adapter ligation mix to the dA-tailed DNA, but the presence of a precipitate did not necessarily indicate failure of ligation of the sequencing adapter to target molecule ends.

### AMPure XP Bead Purification

This step removed excess un-ligated adapters and other short DNA fragments, and the library was concentrated and buffer-exchanged in preparation for sequencing. Agencourt AMPure XP beads (Beckman Coulter, Brea, CA) were brought to RT and resuspended by vortexing. Long Fragment Buffer (LFB; Oxford Nanopore Technologies, Oxford, UK), SPRI Dilution Buffer (SDB; Oxford Nanopore Technologies, Oxford, UK), and Elution Buffer (EB; Oxford Nanopore Technologies, Oxford, UK) were thawed. Short Fragment Buffer (SFB; Oxford Nanopore Technologies, Oxford, UK), rather than LFB, was used to retain DNA fragments shorter than 3 kb. One volume (80 µl) of SDB was to the ligation reaction, and the reaction was mixed gently by flicking the tube. Next, 0.3X volume (48 µl) of AMPure XP beads was added to the ligation reaction. The volume of beads used was calculated based on the volume after the addition of SDB (160 µl). If using a tiling or single-cut strategy was used, samples were pooled together into a single tube following the addition of SDB, and 0.3X volume (96 µl) of AMPure XP beads was added to the ligation reaction. The reaction was mixed gently by inversion, and incubated for 10 min at RT without agitation or pipetting. The reaction was spun down quickly and pelleted on a magnet. The supernatant was pipetted off with the tube kept on the magnet.

The beads were washed by adding 250 µl LFB or SFB, depending on the size of the target molecule. Beads were resuspended in the wash buffer by flicking the tube, then the tube was returned to the magnetic rack and the beads were pelleted. The supernatant was removed and discarded, and the wash procedure was repeated. Following the second wash, the tube was spun down, returned to the magnet, and any residual supernatant was pipetted off. The pellet was dried for approximately 30 seconds (pellets should not be overdried to the point of cracking).

The tube was removed from the magnet, and the pellet was resuspended in 13 µl EB and incubated at RT for 10 min. For fragments > 30 kb, elution time was increased to 30 minutes. Beads were re-pelleted on the magnet until the eluate was clear and colorless. Twelve microliters of eluate was removed and pipetted into a clean 1.5 ml DNA LoBind tube. A Qubit^{®} (ThermoFisher Scientific, Waltham, MA) fluorometric dsDNA BR (broad range) assay was performed using 1 µl of the prepared library. The prepared library was ready to be loaded onto a flow cell; if the prepared library was not immediately loaded onto a flow cell for sequencing, it was stored at 4°C for 24hrs, or -80°C for > 24hrs.

### sgRNA selection

Unique sgRNAs for nanopore Cas9-targeted sequencing were designed upstream and downstream of the region to be sequenced, avoiding any SNPs. Alternatively, if a transgene or insertion genome location was unknown, sgRNAs were designed to a known unique sequence within the suspected insertion. In this and subsequent examples, that unique sequence was/is often Cre. However, in options, sgRNAs may be targeted against any unique nucleotide sequence in the insertion as long as that sequence does not exist in the host genome (including but not limited to Cre; fluorescent proteins such as green fluorescent protein (GFP), red fluorescent protein (RFP), blue fluorescent protein (BFP), yellow fluorescent protein (YFP), or any other fluorescent proteins; luciferase; FLAG; a vector backbone; or a specific gene sequence). In options, sgRNAs may also be targeted to a known nucleotide sequence not previously defined and/or without known biological relevance.

### Sequencing

As previously described [Lesbirel, S. et al., New England Biolabs Expressions 2021, Issue 1], samples were sequenced on MinION R9.4.1 flow cells for 24 hours on either MinION MK1B or GridION Mk1 (Oxford Nanopore Technologies, Oxford, UK). Samples were run as single runs or multiplexed with up to four targets. Flow cells were reused two to four times after washing every 24 hours according to manufacturer's instructions (Flow Cell Wash Kit, Oxford Nanopore Technologies, Oxford, UK).

### Long-read targeted sequencing analysis and insertion/editing region reconstruction

### LORETA bioinformatics pipeline

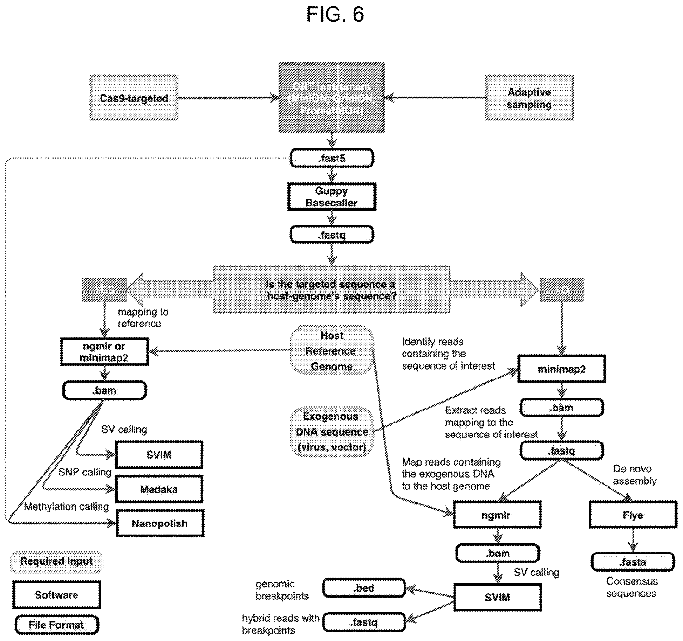

As illustrated in FIG. 6, a long-read targeted sequencing analysis (LORETA) bioinformatics pipeline was designed to process long-read sequencing data generated by different targeting sequencing strategies, including CRISPR-Cas9-targeted sequencing as described above and below herein and in silico adaptive sampling. For CRISPR-Cas9-targeted sequencing, dual- or single-cut libraries were generated as described above and below herein to isolate a region or regions of interest from a subject's genome, and then sequenced with a long-read sequencing method such as MinION, GridION, or PromethION nanopore sequencing (Oxford Nanopore Technologies, Oxford, UK). For in silico adaptive sampling, enrichment was performed in real-time during long-read sequencing, because a software interface was used to permit only DNA fragments from the genomic region of interest to be sequenced.

LORETA provided optional parameters for analysis of two kinds of targeted sequencing experiments, depending on the origin of the targeted sequence: 1) targeting of a specific region of a known genome (reference sequence) or 2) targeting of exogenous DNA inserted into a known genome (reference sequence).

In the first case, targeting of a specific region of a reference genome, LORETA first aligned nanopore reads to the reference genome using minimap2 (github/lh3/minimap2) or NGMLR [Sedlazeck, F.J., et al., Nat. Meth. 15, 461-468 (2018)], then called structural variants in the region(s) of interest using SVIM [Heller and Vingron, Bioinformatics 35(17), 2907-2915 (2019)] and called single nucleotide polymorphisms (SNPs) and small INDELS in the region(s) of interest using medaka (github.com/nanoporetech/medaka). LORETA also performed DNA modification analyses (5-methylcytosine (5-mC)) on regions of interest using Nanopolish (github.com/jts/nanopolish).

In the second case, targeting of exogenous DNA inserted into a known genome, LORETA identified any integration event, including but not limited to those produced by genome editing experiments (such as CRISPR- or TALEN-mediated genome editing) and viral vector integrations. To detect integration events or any other non-reference insertions, LORETA first used minimap2 to map nanopore reads or fastq reads from query samples to an indexed set of sequences of interest (including but not limited to adenoviral (Ad) vectors, transgenes, regulatory cassettes, etc.). Reads containing the sequence of interest were then mapped to the reference (host) genome using NGMLR. Next, SVIM was applied to identify both reads with embedded insertions and the host genome's breakpoints, which in the case of an integration event corresponded to genomic sites of vector integration. Reads that aligned with the vector index and the host genome and showed evidence of insertions according to SVIM were classified as "hybrid" reads. Reads that aligned with the sequence of interest were also used to reconstruct the inserted sequences using a de novo assembly approach (Flye) [Kolmogorov et al., Nat. Biotech. 37(5), 540-546 (2019)], which further allow the identification of both in-tandem integration events and episomal sequences.

### Results

### Cas9 dual-cut library

If the location of an insertion or a region of interest under investigation was known, a library was constructed using two sgRNAs targeting the flanking regions, as illustrated in FIG. 7A and as previously described [Lesbirel, S. et al., New England Biolabs (2021), Issue 1; Gilpatrick, T. et al., Nat. Biotech. 38, 433-438 (2020)]. Both sgRNAs were used in the same reaction, and the resulting library was enriched for the region of interest in a single library preparation.

A Cas9 dual-cut library strategy was employed for rapid strain comparison to investigate sequence variation at the MX1 locus in its entirety by targeting 2 kb up- and down-stream in both the common laboratory strain C57BL/6J (FIG. 7B) and a wild-derived strain CAST/EiJ (FIG. 7C). The resulting data confirmed a known 3.5 kb deletion in C57BL/6J, spanning exons 8 to 12. The deletion appeared as an insertion in the CAST/ EiJ alignment because the mouse reference was constructed with C57BL/6J (FIG. 7C, indicated by arrow). The resulting capture sequencing generated 80X coverage over a 22.5 kb region in CAST/ EiJ and 230X coverage over a 19 kb region in C57BL/6J.

A Cas9 dual-cut library strategy was also employed to validate the integrity of targeted mutations within multiple mouse strains (Samples 1 and 8). In Sample 8, in which exon 4 of a gene of interest was floxed, sgRNAs were designed up and downstream of the floxed exon to excise a 5 kb fragment. 70X coverage of the region of interest was obtained, and two unexpectedly large insertions were detected (indicated in purple in FIG. 7D). One insertion was 180 bp and another was 80 bp. The larger-than-expected insertions were suspected to be a result of plasmid DNA integration along with the loxP sequence. In Sample 1, significantly longer than expected reads were obtained (up to 95 kb) from a targeted region of 13 kb (FIG. 7E) at the terminal end of a gene of interest, covering exons 25-29. The model in question was subject to targeted mutagenesis within this region. Subsequent sequencing yielded a mean of 100X coverage over the target region whilst generating reads 13-95 kb in length. Larger insertions than expected were revealed, indicating the requirement for further investigation into this region. Cas9 dual-cut targeted sequencing saved considerable time, because traditional Sanger-based methods did not yield any insights into this particular locus condition (data not shown).

### Cas9 single-cut library

If an insertion location was unknown, two independent libraries were constructed and pooled prior to sequencing. sgRNAs were designed against an inserted sequence not endogenously present in the host genome; non-limiting examples of inserted sequences include a transgene containing GFP or Cre. One sgRNA was designed against the sense strand of the insert, generating 5' to 3' reads; a second sgRNA was designed against the antisense strand of the insert resulting in 3' to 5' reads (FIGs. 8A-B). Each sgRNA was used to construct a single library, with one sgRNA per library (FIG. 8B).

Multiple promoter-driven Cre mouse lines were analyzed using a single-cut Cas9 library preparation strategy to assess its ability to identify genomic insertion sites. FIG. 8C illustrates an example of identification of an insertion comprising Cre driven by a Sox2 promoter. Other transgenic lines analyzed included MX1-Promoter-Cre (FIG. 9C), Camk2a-Promoter-Cre (FIG. 9D), Tek-Promoter-Cre (FIG. 9E), and hGH-Promoter-Cre (FIG. 9F). MX1-Promoter-Cre transgene insertions were identified within the 5' region of Micu1 on chromosome (Chr) 10 (chr10:59,747,198-59,749,726), with 164 on-target reads ranging from 5-133kb (FIG. 9C, upper and lower panels). LORETA-based analysis of the on-target sequence reads enabled a 74 kb reconstruction of the transgene insertion region, revealing at least four copies of MX1-Promoter-Cre (FIG. 9C, lower panel). Camk2a-Promoter-Cre transgene insertions were identified on Chr 17 (chr17:55,246,762-55,249,924), using 536 Cre containing reads (FIG. 9D, upper and lower panels). LORETA-based analysis and reconstruction of the 43.4 kb transgene insertion region revealed multiple integrations of the transgene cassette coupled with an inversion (FIG. 9D, lower panel). Tek-Promoter-Cre transgene insertions disrupting five genes including Mtrr, Fastkd3, and Adcy2 were identified on Chr 13 (chr13:68,459,931-68,701,276) with 825 on-target reads (FIG. 9E, upper and lower panels). LORETA-based analysis and reconstruction of the 75 kb transgene insertion region identified at least 12 copies of the Tek-Promoter-Cre transgene (FIG. 9E, lower panel).

This strategy enabled identification of transgene insertion locations and revealed complex structural information. Additionally, simultaneous validation of CRISPR modifications and identification of off-target or unwanted integrations such as BAC constructs or plasmid backbone was performed (FIG. 8D, FIG. 9F).

### dCas9:Cas9 single-cut library

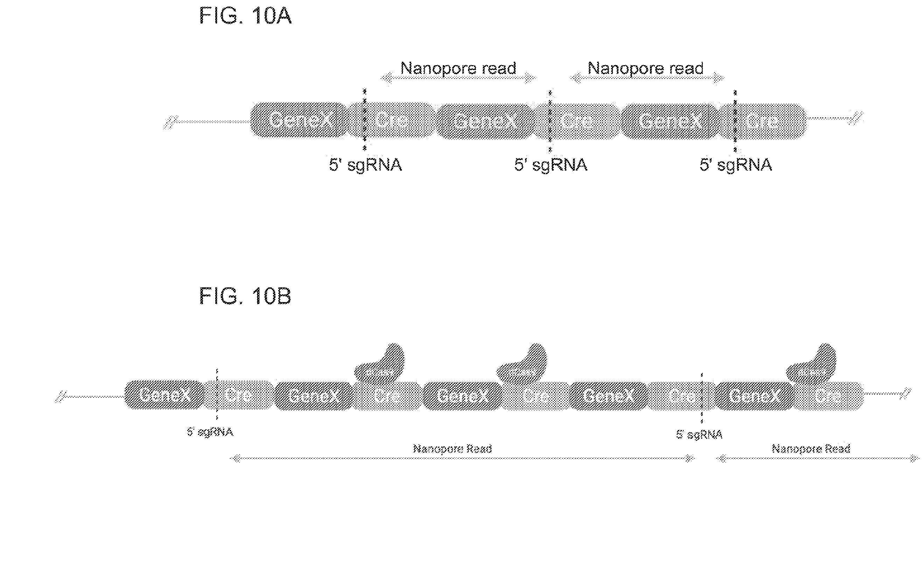

Experiments demonstrated that the majority of transgenic and CRISPR-generated organisms had multiple insertions due to concatemerization of the insertion cassette (see, for example, FIG.9C-E). That concatemerization caused a "short read" problem in long read library preparation because multiple cuts were generated along the concatemerized cassette, thus resulting in over-representation of the transgenic cassette and reduced read length, with concomitant loss of locational and/or structural information (FIG. 10A). The read length distribution problem is also seen in the shape of the violin plots in FIG. 9B, with bulges toward the central portions of their respective distributions that tapered into long, narrow tails over the remainders of their respective distributions.

It was hypothesized that including dCas9 in the initial Cas9-sgRNA cleavage reaction would minimize the "short read" problem, because dCas9-sgRNA complexes would mask binding sites in the concatemerized cassette, resulting in fewer cuts and longer read lengths (FIG. 10B). Experiments were performed using a 1:1 or a 3:1 dCas9:Cas9 ratio in the cleavage reaction. dCas9-sgRNA RNPs were prepared according to Cas9-sgRNA preparation as described above herein. Once dCas9-sgRNA:Cas9-sgRNA cleavage and dA-tailing were complete, the reaction was treated with Proteinase K for 15 min at 56°C followed by an equal volume AMPure bead cleanup. Cleaved DNA was eluted in 10 mM Tris pH8 and adaptor ligation was performed as described above herein.

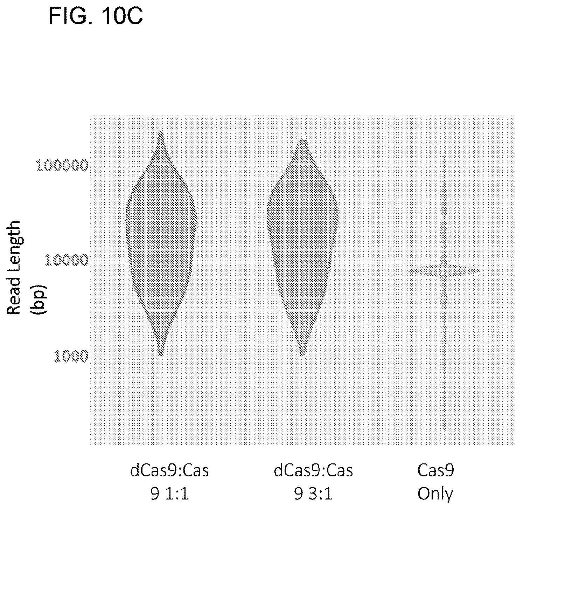

The addition of dCas9 during cleavage reduced the number of reads (Table 6), but increased on-target read length (compare shapes of violin plot distributions in FIG. 10C with shapes in FIG. 9B; Table 6), and increased the number of on-target breakpoint reads.

**Table 6. Addition of dCsas9 during cleavage reduces number of reads**

| **Category** | **1:1 (dCas9:Cas9)** | **3:1 (dCas9:Cas9)** | **Cas9 only** |
|---|---|---|---|
| Mean read length (bases) | 24,002.20 | 22,183.00 | 9779.20 |
| Mean read quality | 11.5 | 11.1 | 12.9 |
| Median read length (bases) | 19,765.00 | 8,988.00 | 7,890.00 |
| Median read quality | 11.8 | 10.2 | 13.2 |
| Number of reads | 20 | 11 | 217 |
| Read length N50 (bases) | 45,109.00 | 47,940.00 | 7,926.00 |
| Total bases | 480,043.00 | 244,013.00 | 2,122,089.00 |

Therefore, the dCas9:Cas9 cleavage strategy successfully addressed the short read problem and enabled improved identification of genomic insertion location(s) for transgenic samples. Such improved identification further enabled investigators to reconstruct insertion regions with increased accuracy.

### LORETA bioinformatics pipeline and analysis

Overall, LORETA provided an all-in-one solution for processing, analysis, and interpretation of long-read targeted sequencing data produced by different Cas9 library preparation strategies and sequencing strategies. For experiments targeting a specific region of a known genome, LORETA reported: basic QC of the sequenced reads (including Phred scores, read length distribution, and N50, etc.), a target and off-target report (efficiency), a structural variation report, a SNPs and INDELs report, methylation calls (if requested), a consensus sequence (if requested), and putatively affected genes or regulatory regions. In the case of exogenous DNA inserted into a known genome (including but not limited to vector integration assays and genome-editing experiments) LORETA reported: basic QC of the sequenced reads (including Phred scores, read length distribution, N50, etc.), the number of integration/insertion events and their genomic coordinates (accompanied by a genome browser picture), the number of reads supporting the integration event, putatively affected genes or regulatory regions, and consensus sequence(s) of the integrated and/or the episomal sequences.

### Example 2. dCas9:Cas9 single-cut library modified protocol

### Materials and Methods

Materials and methods are as disclosed in Example 1, except as described below.

### Results

Because target DNA loss was observed during AMPure bead cleanup, further experiments are performed to advance dCas9 single-cut library development, using biotin-ProteinaseK immobilized onto streptavidin beads. ProK is immobilized on streptavidin magnetic beads and a dCas9 single-cut library or libraries are prepared as disclosed in Example 1. Once dCas9-sgRNA:Cas9-sgRNA cleavage and dA-tailing are complete, the reaction is incubated with the strep-ProK beads for 5-60 minutes at 50-60oC. The beads+gDNA are pelleted on a magnetic rack and the supernatant removed and used in the next phase of the library preparation. Cleaved DNA is eluted in 10 mM Tris pH 8 and adaptor ligation is performed as described above herein.

This strategy removes the need for an AMPure cleanup step, thereby reducing the amount of target DNA loss and increasing sequencing yield in comparison to results obtained using AMPure XP bead cleanup. Some studies include an increase in target read length resulting in a reduction in gDNA loss when removing the ProK from the reaction mix. Sequence analysis and reconstruction is performed with the LORETA bioinformatics pipeline as described above herein.

### Example 3. Quality control and surveillance of integration and/or editing events

Options of the disclosure described in examples above herein may be used in a wide variety of research and clinical applications for rapid confirmation of integration and/or editing events in a subject and identification of any off-target integration events.

To characterize transgene integration in one or more subjects, including but not limited to animal or plant models, an HMW gDNA sample is obtained from the one or more subjects, and a Cas9 dual-cut, single-cut, or dCas9:Cas9 single-cut library or libraries for long-read targeted sequencing are prepared as described above herein, sequenced using nanopore technology, and analyzed using the LORETA bioinformatics pipeline. Location and structural data are obtained for each integration region, including off-target integration events. One or more subjects are selected for future experimental and/or propagation applications on the basis of that location and structural data. For example, transgene integrations in mice are characterized, and the mice selected for future use are those with integration events that do not disrupt endogenous genetic loci and with the fewest off-target events present.

Such characterization of integration and/or editing events with options of the disclosure is further used as a quality control (QC)/surveillance mechanism for model organism generation and for gene therapy. Characterization data generated are compared to identify integration hotspots or frequently observed editing errors, and other potentially confounding or deleterious phenomena such as inversions, deletions, chromosome breakage, inversions, and translocations. Comparison of characterization data allows not only validation of individual subjects, but broader investigation into the performance of integration or editing tools and strategies being used.

### Example 4. Precision cytogenetics, genetic diseases, and cancer genome analysis

Options of the disclosure as disclosed in Examples above herein are used in research and clinical applications including molecular karyotyping, diagnosis of genetic diseases, candidate identification for undiagnosed genetic diseases, and cancer genome analysis.

An HMW DNA sample is obtained from one or more subjects, and Cas9 dual-cut libraries for long-read targeted sequencing are prepared as described above herein. If the one or more subjects are not transgenic, sgRNAs are designed against known unique sequences in the host genome. Libraries are sequenced using nanopore technology, and analyzed using the LORETA bioinformatics pipeline. Location and structural data are obtained for each region of interest, and analyzed for events such as such as mutations inversions, deletions, chromosome breakage, inversions, translocations, episomal DNA, and altered epigenetic modifications.

In some studies single cut or dCas9 strategies are used to assess transgenic subjects, a non-limiting examples of which is for transgenic model quality control. In certain studies single cut or dCas9 strategies are used in methods of assessing efficacy of gene therapy and for gene therapy surveillance.

All definitions, as defined and used herein, should be understood to control over dictionary definitions, and/or ordinary meanings of the defined terms.

The indefinite articles "a" and "an," as used herein in the specification and in the claims, unless clearly indicated to the contrary, should be understood to mean "at least one." The phrase "and/or," as used herein in the specification and in the claims, should be understood to mean "either or both" of the elements so conjoined, i.e., elements that are conjunctively present in some cases and disjunctively present in other cases. Other elements may optionally be present other than the elements specifically identified by the "and/or" clause, whether related or unrelated to those elements specifically identified, unless clearly indicated to the contrary.

## Claims

1. A method of identifying target-specific genetic and epigenetic information in a genomic region containing a DNA sequence of interest, comprising:
(a) extracting an ultralong DNA molecule from a biological sample, wherein the ultralong DNA molecule is at least 500 kb in length;
(b) fragmenting the extracted ultralong DNA molecule to produce DNA molecule fragments, wherein one or more of the produced DNA molecule fragments comprises all or a portion of the DNA sequence of interest, and the cleaved fragments' ends are compatible for ligation of sequencing adaptors,
(c) ligating the sequencing adaptors to the cleaved fragments, and
(d) determining the sequences of the ligated cleaved fragments;
wherein the determined sequences identify genetic and epigenetic information in the genomic region containing the DNA sequence of interest, and,
wherein the method further comprises repeating the fragmenting in step (b) two or more times to cover the full sequence of interest.

2. The method of claim 1, wherein a method of determining the sequences comprises a nanopore sequencing means, comprising:
(a) measuring an ionic current when a single-stranded DNA fragment of the extracted ultralong DNA molecule exposed to a voltage passes through a nanopore;
(b) inferring a nucleotide sequence using real-time base calling from raw current signal data,
(c) removing one or more unwanted DNA fragment molecules by reversing the voltage when the unwanted DNA fragment molecules pass across one or more individual nanopores; and
(d) selecting the DNA fragment molecules containing the DNA sequence of interest.

3. The method of claim 1 or claim 2, wherein a means of fragmenting the ultralong DNA comprises an enzymatic method.

4. The method of any one of claims 1-3, wherein the fragmenting means is a targeted-cleaving method.

5. The method of any one of claims 1-4, wherein the biological sample comprises:
(a) a cell,
optionally wherein the DNA of interest is native to the cell or wherein the cell is a host cell and the DNA of interest is exogeneous DNA to the host cell, optionally wherein the exogenous DNA is:
(i) DNA inserted into the host cell;
(ii) episomal DNA in the host cell or DNA integrated into the host genome;
(iii) a transgene in the host cell;
(iv) comprising a unique sequence not present in the host cell's genome; or,
(v) extrachromosomal; or
(b) a body fluid, optionally wherein the body fluid comprises one of: blood, plasma, saliva, urine, lymph, amniotic fluid, cerebrospinal fluid.

6. The method of any one of claims 1-5, wherein the DNA of interest comprises a predetermined DNA sequence or a DNA sequence positioned at preselected genomic coordinates obtained from a reference genome assembly.

7. The method of claim 4, wherein the targeted cleaving comprises:
(i) binding a plurality of preselected Cas9 sgRNAs to the extracted ultralong DNA molecule, where the specificity of the binding is based on the DNA sequences of interest;
(ii) contacting the bound preselected Cas9 sgRNAs with a plurality of one or more Cas9 enzymes; wherein the preselected Cas9 sgRNAs bound to the extracted ultralong DNA molecule each bind a Cas9 enzyme, forming a plurality of Cas9/sgRNA complex sites in the ultralong DNA molecule; and
(iii) cutting the ultralong DNA molecule at the Cas9/sgRNA complexes thereby producing the DNA molecule fragments, wherein a number and position of the cuts are determined by the preselected Cas9 sgRNAs, and wherein a means for the cutting is a CRISPR/cas9 cutting method and the DNA fragments produced by the cutting comprise termini capable of ligation by the sequence adaptors.

8. The method of claim 4, wherein the targeted cleaving comprises:
(i) binding a plurality of preselected Cas9 sgRNAs to the extracted ultralong DNA molecule;
(ii) contacting the bound preselected Cas9 sgRNAs with a plurality of a non-endonuclease-deficient Cas9 enzyme and a plurality of an endonuclease-deficient Cas9 enzyme (dCas9); wherein the preselected Cas9 sgRNAs bound to the extracted ultralong DNA molecule each bind either a Cas9 enzyme or a dCas9 enzyme, forming Cas9/sgRNA complex sites and dCas9/sgRNA complex sites respectively in the ultralong DNA molecule; and
(iii) cutting the ultralong DNA molecule at the Cas9/sgRNA complex producing the one or more DNA molecule fragments, wherein increasing a ratio of dCas9/sgRNA complex sites: Cas9/sgRNA complex sites in the ultralong DNA molecule decreases the number of cuts to the ultralong DNA molecule and the number of the produced DNA molecule fragments, and decreasing a ratio of dCas9/sgRNA complex sites: Cas9/sgRNA complex sites in the ultralong DNA molecule increases the number of cuts to the ultralong DNA molecule and the number of the produced DNA molecule fragments
optionally further comprising preselecting the ratio of the dCas9/sgRNA complex sites: Cas9/sgRNA complex sites in the ultralong DNA molecule thereby preselecting a length of the produced DNA molecule fragments.

9. The method of claim 7 or claim 8, wherein:
(a) the preselected sgRNAs bind the DNA sequences of interest;
(b) the preselected sgRNAs are capable of binding a sequence contiguous with one or both ends of the DNA sequences of interest; or,
(c) the preselected sgRNAs bind at one or more of: at positions (1) outside the DNA sequence of interest; (2) inside the DNA sequence of interest; and inside and outside the DNA sequence of interest.

10. The method of any one of claims 1-9, further comprising ligating one or more sequencing adaptors to the ultralong DNA molecule fragments.

11. The method of any one of claims 1-10, wherein a means of the sequencing comprises a nano pore sequencing method.

12. The method of any one of claims 1-11, wherein the ultralong DNA molecule is between 1 kb and 500 kb in length, or wherein the ultralong DNA molecule is at least 500 kb in length.

13. The method of claim 1, wherein a means for the extracting comprises:
(a) heating a preselected amount of cells to about 50-60°C in the presence of proteinase K and RNase A;
(b) an alcohol-based precipitation of high molecular weight DNA; or,
(c) a non-alcohol-based precipitation of high molecule weight DNA.

14. The method of any of claims 1-13, further comprising comparing the determined ligated cleaved fragments with one or more reference sequence(s) and identifying a presence or absence of one or more differences in the determined ligated cleaved fragments and the reference sequences, wherein the comparison identifies one or more of a genetic alteration of: integration, insertion, deletion, inversion, translocations, and DNA modifications in the genomic region containing the DNA sequence of interest,
optionally wherein the reference sequence comprises the genomic region containing the DNA sequence of interest in a wild-type cell.

15. The method of any one of claims 5-14, wherein the cell from which the ultralong DNA molecule is extracted is:
(a) a mammalian cell, optionally a mouse cell, further optionally wherein the mouse cell is a blood cell, optionally a plasma cell; or
(b) a plant cell; or
(c) from a mouse model of a disease or condition; or
(d) a genetically engineered cell.

16. A method of assessing efficacy of a means of introducing a candidate genetic modification in a cell, the method comprising:
assessing in a cell treated to introduce a candidate genetic modification in the cell, the sequences of the ligated cleaved fragments determined in claim 1(d); and
identifying the presence or absence of the candidate genetic modification in the assessed determined sequences, wherein the presence of the candidate genetic modification confirms the efficacy of the means of introducing the candidate genetic modification in the cell.

17. The method of claim 16, wherein the cell is
(a) a mammalian cell, optionally wherein the cell is from a mouse model of a disease or condition; or
(b) a plant cell; or
(c) a genetically engineered cell.

## Patentansprüche

1. Verfahren zum Identifizieren von zielspezifischen genetischen und epigenetischen Informationen in einer genomischen Region, die eine DNA-Sequenz von Interesse enthält, umfassend:
(a) Extrahieren eines ultralangen DNA-Moleküls aus einer biologischen Probe, wobei das ultralange DNA-Molekül mindestens 500 kb lang ist;
(b) Fragmentieren des extrahierten ultralangen DNA-Moleküls, um DNA-Molekülfragmente zu erzeugen, wobei eines oder mehrere der erzeugten DNA-Molekülfragmente die gesamte oder einen Abschnitt der DNA-Sequenz von Interesse umfassen und die Enden der gespaltenen Fragmente für die Ligation von Sequenzierungsadaptoren kompatibel sind,
(c) Ligieren der Sequenzierungsadaptoren an die gespaltenen Fragmente, und
(d) Bestimmen der Sequenzen der ligierten gespaltenen Fragmente;
wobei die bestimmten Sequenzen genetische und epigenetische Informationen in der genomischen Region identifizieren, die die DNA-Sequenz von Interesse enthält,
und,
wobei das Verfahren ferner die zwei- oder mehrmalige Wiederholung der Fragmentierung in Schritt (b) umfasst, um die vollständige Sequenz von Interesse abzudecken.

2. Verfahren nach Anspruch 1, wobei ein Verfahren zum Bestimmen der Sequenzen ein Nanoporensequenzierungsmittel umfasst, umfassend:
(a) Messen eines Ionenstroms, wenn ein einzelsträngiges DNA-Fragment des extrahierten ultralangen DNA-Moleküls, das einer Spannung ausgesetzt ist, durch eine Nanopore läuft;
(b) Ableiten einer Nukleotidsequenz unter Verwendung von Echtzeitbasenbestimmung aus rohen Stromsignaldaten,
(c) Entfernen eines oder mehrerer unerwünschter DNA-Fragmentmoleküle durch Umkehrung der Spannung, wenn die unerwünschten DNA-Fragmentmoleküle eine oder mehrere individuelle Nanoporen passieren; und
(d) Auswählen der DNA-Fragmentmoleküle, die die DNA-Sequenz von Interesse enthalten.

3. Verfahren nach Anspruch 1 oder 2, wobei ein Mittel zur Fragmentierung der ultralangen DNA ein enzymatisches Verfahren umfasst.

4. Verfahren nach einem der Ansprüche 1 bis 3, wobei das Fragmentierungsmittel ein gezieltes Spaltungsverfahren ist.

5. Verfahren nach einem der Ansprüche 1 bis 4, wobei die biologische Probe Folgendes umfasst:
(a) eine Zelle, wobei die DNA von Interesse optional in der Zelle nativ ist oder wobei die Zelle eine Wirtszelle ist und die DNA von Interesse eine exogene DNA für die Wirtszelle ist, wobei die exogene DNA optional Folgendes ist:
(i) DNA, die in die Wirtszelle eingeführt wird;
(ii) episomale DNA in der Wirtszelle oder DNA, die in das Wirtsgenom integriert ist;
(iii) ein Transgen in der Wirtszelle;
(iv) umfassend eine einzigartige Sequenz, die im Genom der Wirtszelle nicht vorhanden ist; oder,
(v) extrachromosomal; oder
(b) einen Körperfluid, wobei das Körperfluid optional eines von Folgendem umfasst: Blut, Plasma, Speichel, Urin, Lymphe, Fruchtwasser, Zerebrospinalflüssigkeit.

6. Verfahren nach einem der Ansprüche 1 bis 5, wobei die DNA von Interesse eine vorbestimmte DNA-Sequenz oder eine DNA-Sequenz umfasst, die an vorgewählten genomischen Koordinaten positioniert ist, die aus einer Referenzgenombaugruppe erhalten wurden.

7. Verfahren nach Anspruch 4, wobei die gezielte Spaltung Folgendes umfasst:
(i) Binden einer Vielzahl von vorausgewählten Cas9 sgRNAs an das extrahierte ultralange DNA-Molekül, wobei die Spezifität der Bindung auf den DNA-Sequenzen von Interesse basiert;
(ii) Kontaktieren der gebundenen vorausgewählten Cas9 sgRNAs mit einer Vielzahl von einem oder mehreren Cas9-Enzymen; wobei die vorausgewählten Cas9 sgRNAs, die an das extrahierte ultralange DNA-Molekül gebunden sind, jeweils ein Cas9-Enzym binden und eine Vielzahl von Cas9/sgRNA-Komplexstellen in dem ultralangen DNA-Molekül bilden; und
(iii) Schneiden des ultralangen DNA-Moleküls an den Cas9/sgRNA-Komplexen, wodurch die DNA-Molekülfragmente erzeugt werden, wobei eine Anzahl und Position der Schnitte durch die vorgewählten Cas9 sgRNAs bestimmt werden und wobei ein Mittel für das Schneiden ein CRISPR/cas9-Schneideverfahren ist und die durch das Schneiden erzeugten DNA-Fragmente Termini umfassen, die zur Ligation durch die Sequenzadaptoren fähig sind.

8. Verfahren nach Anspruch 4, wobei die gezielte Spaltung Folgendes umfasst:
(i) Binden einer Vielzahl von vorgewählten Cas9 sgRNAs an das extrahierte ultralange DNA-Molekül;
(ii) Kontaktieren der gebundenen vorausgewählten Cas9 sgRNAs mit einer Vielzahl eines Cas9-Enzyms ohne Endonuklease und einer Vielzahl eines Cas9-Enzyms mit Endonukleasenmangel (dCas9); wobei die an das extrahierte ultralange DNA-Molekül gebundenen vorausgewählten Cas9 sgRNAs jeweils entweder ein Cas9-Enzym oder ein dCas9-Enzym binden und Cas9/sgRNA-Komplexstellen bzw. dCas9/sgRNA-Komplexstellen in dem ultralangen DNA-Molekül bilden; und
(iii) Schneiden des ultralangen DNA-Moleküls am Cas9/sgRNA-Komplex, wodurch das eine oder mehrere DNA-Molekülfragmente erzeugt werden, wobei ein Verhältnis von dCas9/sgRNA-Komplexstellen erhöht wird: Cas9/sgRNA-Komplexstellen im ultralangen DNA-Molekül verringert die Anzahl der Schnitte in das ultralange DNA-Molekül und die Anzahl der erzeugten DNA-Molekülfragmente und verringert ein Verhältnis von dCas9/sgRNA-Komplexstellen: Cas9/sgRNA-Komplexstellen in dem ultralangen DNA-Molekül erhöht die Anzahl der Schnitte in das ultralange DNA-Molekül und die Anzahl der erzeugten DNA-Molekülfragmente, was optional ferner die Vorauswahl des Verhältnisses der dCas9/sgRNA-Komplexstellen umfasst: Cas9/sgRNA-Komplexstellen in dem ultralangen DNA-Molekül, wodurch die Länge der erzeugten DNA-Molekülfragmente vorgewählt wird.

9. Verfahren nach Anspruch 7 oder 8, wobei:
(a) die vorausgewählten sgRNAs die DNA-Sequenzen von Interesse binden;
(b) die vorausgewählten sgRNAs in der Lage sind, eine Sequenz zu binden, die mit einem oder beiden Enden der DNA-Sequenzen von Interesse zusammenhängt; oder,
(c) die vorausgewählten sgRNAs an eine oder mehrere der folgenden Positionen binden: an Positionen (1) außerhalb der DNA-Sequenz von Interesse; (2) innerhalb der DNA-Sequenz von Interesse; und innerhalb und außerhalb der DNA-Sequenz von Interesse.

10. Verfahren nach einem der Ansprüche 1 bis 9, ferner umfassend das Ligieren eines oder mehrerer Sequenzierungsadaptoren an die ultralangen DNA-Molekülfragmente.

11. Verfahren nach einem der Ansprüche 1 bis 10, wobei ein Mittel zur Sequenzierung ein Nanoporensequenzierungsverfahren umfasst.

12. Verfahren nach einem der Ansprüche 1 bis 11, wobei das ultralange DNA-Molekül zwischen 1 kb und 500 kb lang ist, oder wobei das ultralange DNA-Molekül mindestens 500 kb lang ist.

13. Verfahren nach Anspruch 1, wobei ein Mittel zum Extrahieren Folgendes umfasst:
(a) Erhitzen einer vorgewählten Menge von Zellen auf etwa 50-60 °C in Gegenwart von Proteinase K und RNase A;
(b) eine auf Alkohol basierende Ausfällung von DNA mit hohem Molekulargewicht; oder,
(c) eine nicht auf Alkohol basierende Ausfällung von DNA mit hohem Molekulargewicht.

14. Verfahren nach einem der Ansprüche 1 bis 13, ferner umfassend das Vergleichen der bestimmten ligierten gespaltenen Fragmente mit einer oder mehreren Referenzsequenz(en) und das Identifizieren des Vorhandenseins oder der Abwesenheit eines oder mehrerer Unterschiede in den bestimmten ligierten gespaltenen Fragmenten und den Referenzsequenzen, wobei der Vergleich eine oder mehrere der folgenden genetischen Veränderungen identifiziert: Integration, Insertion, Deletion, Inversion, Translokationen und DNA-Modifikationen in der genomischen Region, die die DNA-Sequenz von Interesse enthält,
optional wobei die Referenzsequenz die genomische Region umfasst, die die DNA-Sequenz von Interesse in einer Wildtyp-Zelle enthält.

15. Verfahren nach einem der Ansprüche 5 bis 14, wobei die Zelle, aus der das ultralange DNA-Molekül extrahiert wird, eine Zelle ist:
(a) eine Säugetierzelle, optional eine Mauszelle, wobei die Mauszelle ferner optional eine Blutzelle, optional eine Plasmazelle ist; oder
(b) eine Pflanzenzelle; oder
(c) aus einem Mausmodell einer Krankheit oder eines Zustands; oder
(d) eine genetisch manipulierte Zelle.

16. Verfahren zum Bewerten der Wirksamkeit eines Mittels zur Einführung einer genetischen Modifikation in eine Zelle, das Verfahren umfassend:
Bewerten der Sequenzen der ligierten gespaltenen Fragmente, die in Anspruch 1(d) bestimmt wurden, in einer Zelle, die behandelt wurde, um eine genetische Modifikation in die Zelle einzuführen; und
Identifizieren des Vorhandenseins oder der Abwesenheit der genetischen Modifikation des Kandidaten in den untersuchten Sequenzen, wobei das Vorhandensein der genetischen Modifikation des Kandidaten die Wirksamkeit der Mittel zur Einführung der genetischen Modifikation des Kandidaten in die Zelle bestätigt.

17. Verfahren nach Anspruch 16, wobei die Zelle Folgendes ist
(a) eine Säugetierzelle, wobei die Zelle optional von einem Mausmodell einer Krankheit oder eines Zustands stammt; oder
(b) eine Pflanzenzelle; oder
(c) eine genetisch manipulierte Zelle.

## Revendications

1. Procédé d'identification d'informations génétiques et épigénétiques spécifiques à une cible dans une région génomique contenant une séquence d'ADN d'intérêt, comprenant :
(a) l'extraction d'une molécule d'ADN ultra-longue à partir d'un échantillon biologique, dans lequel la molécule d'ADN ultra-longue a une longueur d'au moins 500 kb ;
(b) la fragmentation de la molécule d'ADN ultra-longue extraite pour produire des fragments de molécules d'ADN, dans lequel un ou plusieurs des fragments de molécules d'ADN produits comprennent tout ou une partie de la séquence d'ADN d'intérêt, et les extrémités des fragments clivés sont compatibles pour la ligature d'adaptateurs de séquençage,
(c) la ligature des adaptateurs de séquençage aux fragments clivés, et
(d) la détermination des séquences des fragments clivés ligaturés ;
dans lequel les séquences déterminées identifient des informations génétiques et épigénétiques dans la région génomique contenant la séquence d'ADN d'intérêt, et,
dans lequel le procédé comprend en outre la répétition de la fragmentation à l'étape (b) deux fois ou plus pour couvrir la séquence complète d'intérêt.

2. Procédé selon la revendication 1, dans lequel un procédé de détermination des séquences comprend un moyen de séquençage par nanopores, comprenant :
(a) la mesure d'un courant ionique lorsqu'un fragment d'ADN simple brin de la molécule d'ADN ultra-longue extraite, exposé à une tension, passe à travers un nanopore ;
(b) la déduction d'une séquence nucléotidique à l'aide de l'identification des bases en temps réel à partir des données de signal électrique brutes,
(c) l'élimination d'une ou plusieurs molécules de fragments d'ADN indésirables en inversant la tension lorsque les molécules de fragments d'ADN indésirables traversent un ou plusieurs nanopores individuels ; et
(d) la sélection des molécules de fragments d'ADN contenant la séquence d'ADN d'intérêt.

3. Procédé selon la revendication 1 ou la revendication 2, dans lequel un moyen de fragmentation de l'ADN ultra-long comprend un procédé enzymatique.

4. Procédé selon l'une quelconque des revendications 1 à 3, dans lequel le moyen de fragmentation est un procédé de clivage ciblé.

5. Procédé selon l'une quelconque des revendications 1 à 4, dans lequel l'échantillon biologique comprend :
(a) une cellule, éventuellement dans lequel l'ADN d'intérêt est natif de la cellule ou dans lequel la cellule est une cellule hôte et l'ADN d'intérêt est un ADN exogène à la cellule hôte, éventuellement dans lequel l'ADN exogène est :
(i) un ADN inséré dans la cellule hôte ;
(ii) un ADN épisomique dans la cellule hôte ou un ADN intégré dans le génome hôte ;
(iii) un transgène dans la cellule hôte ;
(iv) comprenant une séquence unique non présente dans le génome de la cellule hôte ; ou
(v) extrachromosomique ; ou
(b) un fluide corporel, éventuellement dans lequel le fluide corporel comprend l'un des éléments suivants : du sang, du plasma, de la salive, de l'urine, de la lymphe, du liquide amniotique, du liquide céphalo-rachidien.

6. Procédé selon l'une quelconque des revendications 1 à 5, dans lequel l'ADN d'intérêt comprend une séquence d'ADN prédéterminée ou une séquence d'ADN positionnée à des coordonnées génomiques présélectionnées obtenues à partir d'un assemblage de génomes de référence.

7. Procédé selon la revendication 4, dans lequel le clivage ciblé comprend :
(i) la liaison d'une pluralité d'ARNg Cas9 présélectionnés à la molécule d'ADN ultra-longue extraite, où la spécificité de la liaison repose sur les séquences d'ADN d'intérêt ;
(ii) la mise en contact des ARNg Cas9 présélectionnés liés avec une pluralité d'une ou plusieurs enzymes Cas9 ; dans lequel les ARNg Cas9 présélectionnés liés à la molécule d'ADN ultra-longue extraite se lient chacun à une enzyme Cas9, formant une pluralité de sites du complexe Cas9/ARNg dans la molécule d'ADN ultra-longue ; et
(iii) la coupure de la molécule d'ADN ultra-longue au niveau des complexes Cas9/ARNg, produisant ainsi les fragments de molécule d'ADN, dans lequel un nombre et une position des coupures sont déterminés par les ARNg Cas9 présélectionnés, et dans lequel un moyen pour la coupure est un procédé de coupure CRISPR/Cas9 et les fragments d'ADN produits par la coupure comprennent des extrémités pouvant être ligaturées par les adaptateurs de séquence.

8. Procédé selon la revendication 4, dans lequel le clivage ciblé comprend :
(i) la liaison d'une pluralité d'ARNg Cas9 présélectionnés à la molécule d'ADN ultra-longue extraite ;
(ii) la mise en contact des ARNg Cas9 présélectionnés liés avec une pluralité d'une enzyme Cas9 non déficiente en endonucléase et une pluralité d'une enzyme Cas9 déficiente en endonucléase (dCas9) ; dans lequel les ARNg Cas9 présélectionnés liés à la molécule d'ADN ultra-longue extraite se lient chacun soit à une enzyme Cas9, soit à une enzyme dCas9, formant respectivement des sites du complexe Cas9/ARNg et des sites du complexe dCas9/ARNg dans la molécule d'ADN ultra-longue ; et
(iii) la coupure de la molécule d'ADN ultra-longue au niveau du complexe Cas9/ARNg produisant l'un ou plusieurs fragments de molécule d'ADN, dans lequel l'augmentation d'un ratio des sites du complexe dCas9/ARNg : les sites du complexe Cas9/ARNg dans la molécule d'ADN ultra-longue réduisent le nombre de coupures dans la molécule d'ADN ultra-longue et le nombre de fragments de molécules d'ADN produits, et diminuent un ratio des sites du complexe dCas9/ARNg : les sites du complexe Cas9/ARNg dans la molécule d'ADN ultra-longue augmentent le nombre de coupures dans la molécule d'ADN ultra-longue et le nombre de fragments de molécules d'ADN produits comprenant en outre éventuellement la présélection du ratio des sites du complexe dCas9/ARNg : les sites du complexe Cas9/ARNg dans la molécule d'ADN ultra-longue, présélectionnant ainsi une longueur des fragments de molécules d'ADN produits.

9. Procédé selon la revendication 7 ou la revendication 8, dans lequel :
(a) les ARNg présélectionnés lient les séquences d'ADN d'intérêt ;
(b) les ARNg présélectionnés sont capables de se lier à une séquence contiguë à une ou aux deux extrémités des séquences d'ADN d'intérêt ; ou
(c) les ARNg présélectionnés se lient à une ou plusieurs des positions suivantes : (1) à l'extérieur de la séquence d'ADN d'intérêt ; (2) à l'intérieur de la séquence d'ADN d'intérêt ; et à l'intérieur et à l'extérieur de la séquence d'ADN d'intérêt.

10. Procédé selon l'une quelconque des revendications 1 à 9, comprenant en outre la ligature d'un ou plusieurs adaptateurs de séquençage aux fragments de molécules d'ADN ultra-longues.

11. Procédé selon l'une quelconque des revendications 1 à 10, dans lequel un moyen du séquençage comprend un procédé de séquençage à nanopores.

12. Procédé selon l'une quelconque des revendications 1 à 11, dans lequel la molécule d'ADN ultra-longue a une longueur comprise entre 1 kb et 500 kb, ou dans lequel la molécule d'ADN ultra-longue a une longueur d'au moins 500 kb.

13. Procédé selon la revendication 1, dans lequel un moyen pour l'extraction comprend :
(a) le chauffage d'une quantité présélectionnée de cellules à environ 50 à 60 °C en présence de protéinase K et de RNase A ;
(b) une précipitation à base d'alcool d'ADN de haut poids moléculaire ; ou
(c) une précipitation sans alcool d'ADN de haut poids moléculaire.

14. Procédé selon l'une quelconque des revendications 1 à 13, comprenant en outre la comparaison des fragments clivés ligaturés déterminés avec une ou plusieurs séquences de référence et l'identification d'une présence ou d'une absence d'une ou plusieurs différences entre les fragments clivés ligaturés déterminés et les séquences de référence, dans lequel la comparaison identifie une ou plusieurs altérations génétiques parmi : l'intégration, l'insertion, la délétion, l'inversion, les translocations et les modifications de l'ADN dans la région génomique contenant la séquence d'ADN d'intérêt, éventuellement dans lequel la séquence de référence comprend la région génomique contenant la séquence d'ADN d'intérêt dans une cellule de type sauvage.

15. Procédé selon l'une quelconque des revendications 5 à 14, dans lequel la cellule à partir de laquelle la molécule d'ADN ultra-longue est extraite est :
(a) une cellule de mammifère, éventuellement une cellule murine, éventuellement en outre dans lequel la cellule murine est une cellule sanguine, éventuellement un plasmocyte ; ou
(b) une cellule végétale ; ou
(c) provient d'un modèle murin d'une maladie ou d'une affection ; ou
(d) une cellule génétiquement modifiée.

16. Procédé d'évaluation de l'efficacité d'un moyen d'introduction d'une modification génétique candidate dans une cellule, le procédé comprenant :
l'évaluation dans une cellule traitée pour introduire une modification génétique candidate dans la cellule, des séquences des fragments clivés ligaturés déterminées dans la revendication 1(d) ; et
l'identification de la présence ou de l'absence de la modification génétique candidate dans les séquences déterminées évaluées, dans lequel la présence de la modification génétique candidate confirme l'efficacité du moyen d'introduction de la modification génétique candidate dans la cellule.

17. Procédé selon la revendication 16, dans lequel la cellule est
(a) une cellule de mammifère, éventuellement dans lequel la cellule provient d'un modèle murin d'une maladie ou d'une affection ; ou
(b) une cellule végétale ; ou
(c) une cellule génétiquement modifiée.
